# EUROPEAN PATENT SPECIFICATION

(11) **EP 3 773 330 B1**
(45) Date of publication and mention of the grant of the patent: **05.03.2025**
(21) Application number: 19778363.2
(22) Date of filing: 29.03.2019
(51) Int. Cl.: A61F 2/02, A61K 38/13, A61K 9/00, A61K 9/16, A61K 9/70, A61F 9/00

(54) **OPHTHALMIC DRUG SUSTAINED RELEASE FORMULATION AND USES FOR DRY EYE SYNDROME TREATMENT**
FORMULIERUNG ZUR VERZÖGERTEN FREISETZUNG OPHTHALMISCHER ARZNEIMITTEL UND VERWENDUNGEN FÜR DIE BEHANDLUNG VON TROCKENEM AUGE
FORMULATION DE MÉDICAMENT OPHTALMIQUE À LIBÉRATION PROLONGÉE ET UTILISATIONS POUR LE TRAITEMENT DU SYNDROME DE L'OEUIL SEC

(30) Priority: 29.03.2018 US 201862650157 P; 30.09.2018 US 201862739320 P; 01.10.2018 US 201862739466 P
(43) Date of publication of application: 17.02.2021
(73) Proprietor: Mati Therapeutics Inc., Austin TX 78701 (US)
(72) Inventor: UTKHEDE, Deepank, Burnaby, British Columbia V5G 4X4 (CA); WISEMAN, David, Burnaby, British Columbia V5G 4X4 (CA); ANDERSON, Koren, Corvallis, Oregon 97330 (US)
(74) Representative: HGF
(86) International application number: PCT/US2019/025025
(87) International publication number: WO 2019/191700

(56) References cited:
- WO-A1-2009/079559
- WO-A2-2004/066980
- WO-A2-2004/066980
- WO-A2-2009/035562
- US-A1- 2006 263 409
- US-A1- 2008 113 027
- US-A1- 2009 318 549
- US-A1- 2010 305 046
- US-B2- 6 953 776
- US-B2- 7 354 574

## Description

This application claims the benefit of U.S. Provisional Patent Application Nos. 62/650,157, filed on 29 March 2018; 62/739,320 filed 30 September 2018; and, 62/739,466 filed on 01 October 2018.

### FIELD OF THE INVENTION

This application pertains to sustained release formulations for topical delivery of ophthalmic drugs to the eye and their uses thereof for methods of treating keratoconjunctivitis sicca (dry eye syndrome).

### BACKGROUND OF THE INVENTION

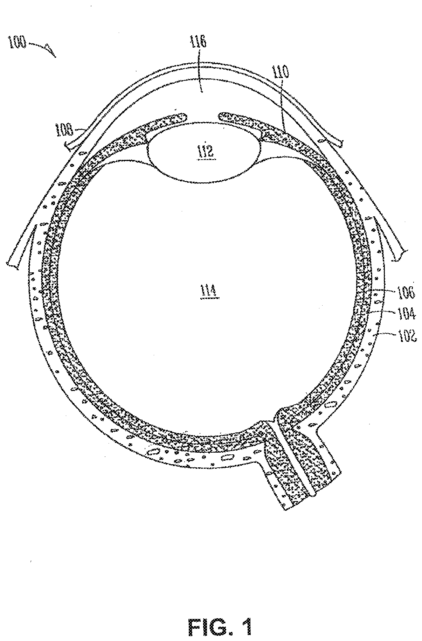

Figures 1-2 illustrate example views of anatomical tissue structures associated with an eye 100. Certain of the anatomical tissue structures shown may be suitable for treatment using the various lacrimal implants and methods discussed herein. The eye 100 is a spherical structure including a wall having three layers: an outer sclera 102, a middle choroid layer 104 and an inner retina 106. The sclera 102 includes a tough fibrous coating that protects the inner layers. It is mostly white except for the transparent area at the front, commonly known as the cornea 108, which allows light to enter the eye 100.

The choroid layer 104, situated inside the sclera 102, contains many blood vessels and is modified at the front of the eye 100 as a pigmented iris 110. A biconvex lens 112 is situated just behind the pupil. A chamber 114 behind the lens 112 is filled with vitreous humor, a gelatinous substance. Anterior and posterior chambers 116 are situated between the cornea 108 and iris 110, respectively and filled with aqueous humor. At the back of the eye 100 is the light-detecting retina 106.

The cornea 108 is an optically transparent tissue that conveys images to the back of the eye 100. It includes a vascular tissue to which nutrients and oxygen are supplied via bathing with lacrimal fluid and aqueous humor as well as from blood vessels that line the junction between the cornea 108 and sclera 102. The cornea 108 includes a pathway for the permeation of drugs into the eye 100.

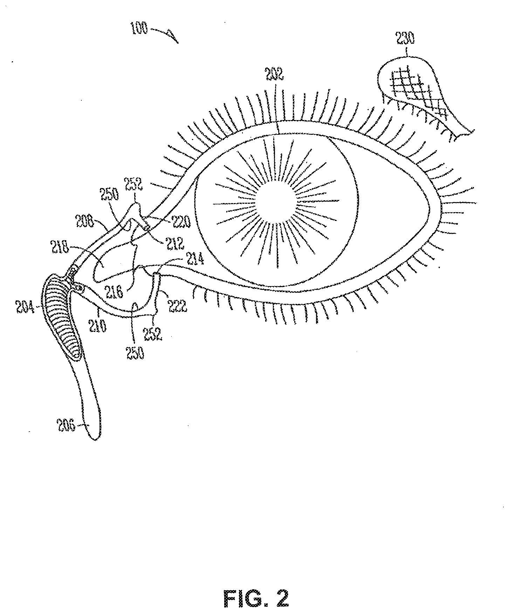

Turing to Figure 2, other anatomical tissue structures associated with the eye 100 including the lacrimal drainage system, which includes a secretory system 230, a distributive system and an excretory system, are shown. The secretory system 230 comprises secretors that are stimulated by blinking and temperature change due to tear evaporation and reflex secretors that have an efferent parasympathetic nerve supply and secrete tears in response to physical or emotional stimulation. The distributive system includes the eyelids 202 and the tear meniscus around the lid edges of an open eye, which spread tears over the ocular surface by blinking, thus reducing dry areas from developing.

The excretory system of the lacrimal drainage system includes, in order of flow, drainage, the lacrimal puncta, the lacrimal canaliculi, the lacrimal sac 204 and the lacrimal duct 206. From the lacrimal duct 206, tears and other flowable materials drain into a passage of the nasolacrimal system. The lacrimal canaliculi include an upper (superior) lacrimal canaliculus 208 and a lower (inferior) lacrimal canaliculus 210, which respectively terminate in an upper 212 and lower 214 lacrimal punctum. The upper 212 and lower 214 punctum are slightly elevated at the medial end of a lid margin at the junction 216 of the ciliary and lacrimal portions near a conjunctival sac 218. The upper 212 and lower 214 punctum are generally round or slightly ovoid openings surrounded by a connective ring of tissue. Each of puncta 212, 214 leads into a vertical portion 220, 222 of their respective canaliculus before turning more horizontal at a canaliculus curvature 250 to join one another at the entrance of the lacrimal sac 204. The canaliculi 208, 210 are generally tubular in shape and lined by stratified squamous epithelium surrounded by elastic tissue, which permits them to be dilated. As shown, a lacrimal canaliculus ampulla 252 exists near an outer edge of each canaliculus curvature 250.

A variety of challenges face patients and physicians in the area of drug delivery, for example, ocular drug delivery. In particular, the repetitive nature of the therapies (multiple injections, instilling multiple eye drop regimens per day), the associated costs, and the lack of patient compliance may significantly impact the efficacy of the therapies available, leading to reduction in vision and many times blindness.

Patient compliance in taking the medications, for example, instilling the eye drops, can be erratic, and in some cases, patients may not follow the directed treatment regime. Lack of compliance can include, failure to instill the drops, ineffective technique (instilling less than required), excessive use of the drops (leading to systemic side effects) and use of non-prescribed drops or failure to follow the treatment regime requiring multiple types of drops. Many of the medications may require the patient to instill them up to 4 times a day.

A conventional method of drug delivery is by topical drop application to the eye's surface. Topical eye drops, though effective, can be inefficient. For instance, when an eye drop is instilled in an eye, it often overfills the conjunctival sac (i.e., the pocket between the eye and the associated lids) causing a substantial portion of the drop to be lost due to overflow of the lid margin and spillage onto the cheek. In addition, a large portion of the drop remaining on the ocular surface can be washed away into and through a lacrimal canaliculus, thereby diluting the concentration of the drug before it can treat the eye. Further, in some cases, topically applied medications have a peak ocular effect within about two hours, after which additional applications of the medications should be performed to maintain the therapeutic benefit.

To compound ocular management difficulty, subjects often do not use their eye drops as prescribed. Noncompliance rates by drop users of 25% and greater have been previously reported. This poor compliance can be due to, for example, forgetfulness or an initial stinging or burning sensation caused by the eye drop and experience by a subject. Instilling eye drops in one's own eye can be difficult, in part because of the normal reflex to protect the eye. Therefore, one or more drops may miss the eye. Older subjects may have additional problems instilling drops due to arthritis, unsteadiness, and decreased vision. Pediatric and psychiatric populations pose difficulties as well.

One promising approach to ocular drug delivery is to place an implant that releases a drug in tissue in or near the eye. However, providing a sustained release of a particular ophthalmic drug at a therapeutic dose over a desired period of time is challenging. Moreover, use of a lacrimal implant provides a limited volume in which to include the drug and a sustained release matrix, wherein elution of the drug must be both relatively constant and at a therapeutic dose over the desired time period. Certain implant compositions suitable for ocular drug delivery are disclosed in WO 2009/079559 A1, WO 2009/035562 A2, WO 2004/066980 A2 and US 7 354 574 B2.

In light of the above, it would be desirable to provide sustained release of certain ophthalmic drugs that overcome at least the above-mentioned shortcomings.

### SUMMARY OF THE INVENTION

Herein are disclosed sustained release formulations for the topical delivery of ophthalmic drugs to the eye, drug inserts and drug delivery systems comprising the formulation, and such compositions for use in the treatment of dry eye.

The present invention relates to a sustained release ophthalmic formulation according to claim 1.

In embodiments are provided a sustained release ophthalmic formulation for topical delivery of an ophthalmic drug, wherein the formulation further comprises a nonionic surfactant, wherein the formulation does not comprise a hydrophilic polymer and the formulation is adapted to release the ophthalmic drug being cyclosporine at therapeutically effective levels each day for a period of about two weeks to about 8 weeks.

In embodiments are provided a sustained release ophthalmic formulation for topical delivery of an ophthalmic drug, wherein the formulation comprises a) at least one hydrophobic polymer; b) a nonionic surfactant; and, c) the ophthalmic drug, wherein the formulation does not comprise a hydrophilic polymer and wherein the hydrophobic polymer is polycaprolactone and is present from about 12.5% (w/w), the nonionic surfactant is polysorbate 80 and is present from about 0 to 22.5% (w/w), and the ophthalmic drug is cyclosporine and is present from about up to 80% (w/w).

In embodiments are provided a sustained release ophthalmic formulation for topical delivery of an ophthalmic drug, wherein the formulation comprises a) at least one hydrophobic polymer; b) a nonionic surfactant; and, c) the ophthalmic drug, wherein the formulation does not comprise a hydrophilic polymer and wherein the hydrophobic polymer is polycaprolactone and is present from 15 to 30% (w/w), the nonionic surfactant is polysorbate 80 and is present from 4.5 to 10% (w/w), and the ophthalmic drug is cyclosporine and is present from 70 to 80% (w/w).

In embodiments are provided sustained release ophthalmic formulation for topical delivery of an ophthalmic drug, wherein the formulation does not comprise a hydrophilic polymer or a nonionic surfactant and the formulation is adapted to release the ophthalmic drug being cyclosporine at therapeutically effective levels each day for a period of about two weeks to about 8 weeks. In embodiments, a first hydrophobic polymer is polycaprolactone and is present from 15 to 30% (w/w), a second hydrophobic polymer is polyvinyl acetate and is present from 0 to 15% (w/w), and the ophthalmic drug is cyclosporine and is present from 70 to 80% (w/w).

The ophthalmic drug being cyclosporine is admixed with one ore more hydrophobic polymers and optionally a nonionic surfactant to form a solid matrix composition, wherein the composition is in the form of a drug core and configured for placement within a lacrimal canaliculus.

In embodiments, the formulations are configured as a medical device including lacrimal implants, punctal plugs, suitable for insertion into the lacrimal canaliculus of the eye. In certain embodiments, the medical device has a substantially cylindrical shape. The formulation further comprises a sheath body disposed at least partially over the matrix.

In embodiments provided herein is a drug insert comprising a present sustained release formulation as a drug core and an impermeable sheath body partially covering the drug core. In embodiments, the drug insert is manufactured by extruding an admixture of drug and polymer (e.g. present sustained release formulation) into the impermeable sheath, optionally cut to a desirable length and optionally sealing one end. In embodiments the drug inserts are cut to a length of about 24.13 mm (0.95 inches) and one end sealed with a medical grade adhesive.

In embodiments, the present drug insert is suitable to be placed in a cavity of a lacrimal implant to form a drug delivery system. In embodiments provided herein is a lacrimal implant comprising a punctal plug comprising a plug body and a drug insert, wherein the insert comprises; a drug core comprising the present sustained release formulation, and an impermeable sheath body partially covering the drug core, wherein the sheath body is configured to provide an exposed proximal end of the drug core in direct contact with tear fluid that releases therapeutic agent to the eye when the drug insert is disposed within a channel of the punctal plug and the punctal plug is inserted into the lacrimal canaliculus of a patient.

In embodiments provided herein, the sustained release formulation, as a medical device, drug insert or drug delivery system, is for use in the treatment of dry eye. In embodiments provided herein is a medical use for delivering a drug for dry eye treatment to the eye, comprising, placing a lacrimal implant through a punctum and into a canalicular lumen of a patient, the implant comprising; a present sustained release ophthalmic formulation, wherein the ophthalmic drug is a cyclosporine and the matrix is configured for delivery of a daily therapeutic amount of cyclosporine for a period of at least 2 weeks and up to 6 months.

### BRIEF DESCRIPTION OF THE DRAWINGS

In the drawings, like numerals describe similar components throughout the several views. Like numerals having different letter suffixes represent different instances of similar components. The drawings illustrate generally, by way of example, but not by way of limitation, various embodiments disclosed herein.
FIG. 1 illustrates an example of anatomical tissue structures associated with an eye, certain of these tissue structures providing a suitable environment in which a lacrimal implant can be used.
FIG. 2 illustrates another example of anatomical tissue structures associated with an eye, certain of these tissue structures providing a suitable environment in which a lacrimal implant can be used.
FIG. 3A provides a perspective view of an implant in accordance with an embodiment of the present invention.
FIG. 3B is a side view of an implant in accordance with an embodiment of the present invention.
FIG. 3C is a side view illustrating the second member and the third member of an implant in accordance with an embodiment of the present invention.
FIG. 3D is a back view of an implant in accordance with an embodiment of the present invention.
FIG. 3E is a cross-sectional view taken about line III(E)-III(E) of FIG. 3D depicting an implant with a bore, in accordance with an embodiment of the present invention.
FIG. 3F is a partially enlarged view of FIG. 3E taken about circle III(F) depicting the second member, the third member and a bore formed in the third member of an implant, in accordance with an embodiment of the present invention.
FIG. 4A provides a perspective view of an implant in accordance with an embodiment of the present invention.
FIG. 4B is a cross-sectional view depicting an implant having a cavity formed in the second member, in accordance with an embodiment of the present invention.
FIG. 4C is a partially enlarged view taken about circle IV(C) of FIG. 4B depicting a cavity in the second member and a bore in the third member of an implant, in accordance with an embodiment of the present invention.
FIG. 5 provides a partial cross-sectional view of an implant in accordance with one embodiment of the present invention.
FIG. 6 provides a partial cross-section view of an implant in accordance with another embodiment of the present invention.
Figure 7 shows elution data of cyclosporine from drug cores manufactured with polycaprolactone (PLC) at a range of 17.5 to 32.5% (w/w); polysorbate 80 (PS80) at a range of 7.5 to 22.5% (w/w); and, cyclosporine at a range of 60 to 67.5% (w/w) over a time period of 67 days. The different ratio of components in the formulations are presented as cyclosporine/Polysorbate 80/polycaprolactone in the Figure. The formulations all show an elution rate of at least 1.5µg/day at day 48 of cyclosporine.
Figure 8 shows the same elution data as Figure 7, but over a shorter time period of 35 days.
Figure 9 shows elution data of cyclosporine from drug cores manufactured with polycaprolactone (PLC) at a range of 14 to 25.5% (w/w); polysorbate 80 (PS80) at a range of 4.5 to 7.5% (w/w); and, cyclosporine at a range of 70 to 80% (w/w/) over a time period of 34 days. The different ratio of components in the formulations are presented as cyclosporine/PS80/PCL. The formulations all show an elution rate above the target (1.5 µg/day) for 34 days of cyclosporine.
Figure 10 shows elution data of cyclosporine from drug cores of different lengths (950 µm to 1100 µm) manufactured with polycaprolactone (PLC) at about 30% (w/w); and, cyclosporine at about 70% (w/w) over a time period of 50 days. The formulations all show an elution rate of at least 1.5µg/day at day 45 of cyclosporine.
Figure 11 shows elution data of cyclosporine from drug cores manufactured with polycaprolactone (PLC) at a range of 15 to 25% (w/w); polyvinyl acetate at a range of 0 to 15% (w/w); and, cyclosporine at a range of 70 to 80% (w/w) over a time period of 60 days. The formulations all show an elution rate of at least 1.5µg/day at day 55 of cyclosporine.
Figure 12 shows elution data of cyclosporine from drug cores manufacture with polycaprolactone (PLC) at a range of 17 to 30% (w/w); polyvinyl acetate at a range of 0 to 5% (w/w); polysorbate 80 at a range of 0 to 3% (w/w) and, cyclosporine at a range of 70 to 75% (w/w) over a time period of 65 days. The formulations all show an elution rate of at least 1.5µg/day at day 54 of cyclosporine.
Figure 13 shows elution data of cyclosporine from drug cores with a length of 1100 µm manufactured with polycaprolactone (PLC) at about 17 or 20% (w/w); polyvinyl acetate at about 5% (w/w), polysorbate 80 at 0 or 3% (w/w), and, cyclosporine at about 75% (w/w) over a time period of 65 days. The formulations all show an elution rate of at least 1.5µg/day at day 60 of cyclosporine.
Figure 14 shows elution data of cyclosporine from drug cores manufacture with polycaprolactone (PLC) at a range of 5 to 20% (w/w); polyvinyl acetate at a range of 5 to 20% (w/w); no polysorbate 80; and, cyclosporine at 75% (w/w) over a time period of 13 days.
Figure 15 shows elution data of cyclosporine from drug cores manufacture with polycaprolactone (PLC) at a range of 5 to 17% (w/w); polyvinyl acetate at a range of 5 to 17% (w/w); polysorbate 80 at 3% (w/w) and, cyclosporine at 75% (w/w) over a time period of 13 days.

### DETAILED DESCRIPTION OF THE INVENTION

### Introduction

Disclosed herein are compositions, devices comprising said compositions and their medical uses for the sustained topical delivery of an ophthalmic drug to an eye, according to the appended claims. In embodiments, the compositions comprise an ophthalmic drug being cyclosporine admixed with one or more hydrophobic polymers and optionally a non-ionic surfactant to form a solid matrix composition, wherein the formulation does not comprise a hydrophilic polymer and the formulation is adapted to release the ophthalmic drug at therapeutically effective levels each day for a period of about two weeks to about 8 weeks. In certain embodiments, the compositions comprise an ophthalmic drug (i.e., cyclosporine) admixed with one or more hydrophobic polymers to form a solid matrix composition, wherein the formulation does not comprise a hydrophilic polymer or a non-ionic surfactant, and the formulation is adapted to release the ophthalmic drug at therapeutically effective levels each day for a period of about two weeks to about 8 weeks.

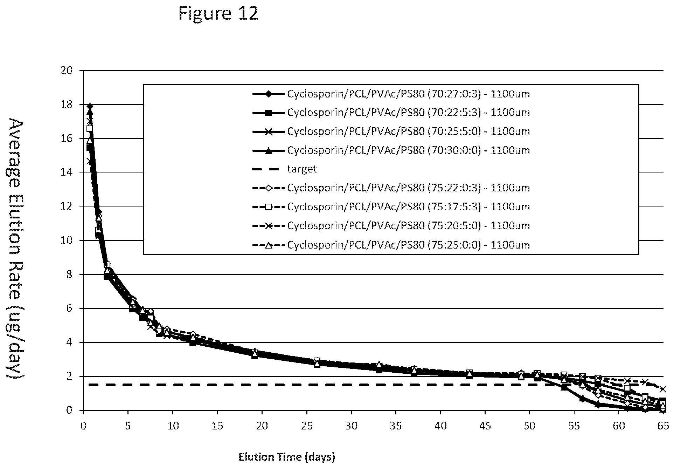

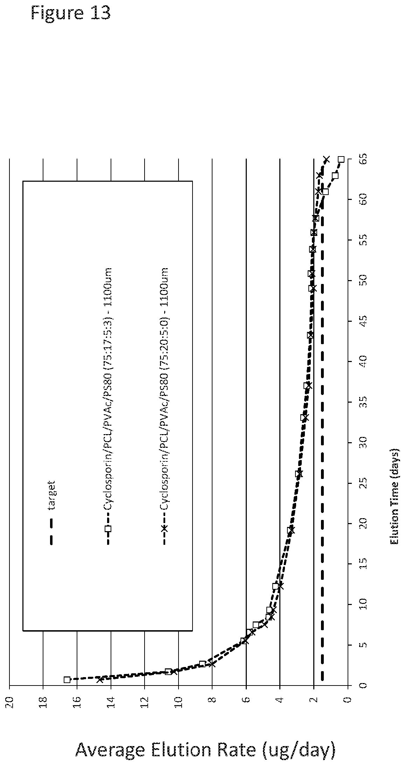

Without wishing to be bound by theory, the removal of hydrophilic polymers and/or nonionic surfactants increase the duration for elution (without negatively impacting an initial burst of drug) of a therapeutic dose (e.g. 1.5µg/day of cyclosporine) of an ophthalmic drug without increasing the overall amount of drug present in the formulation. We have found that a formulation of cyclosporine admixed with a hydrophobic polymer, hydrophilic polymer and a nonionic surfactant demonstrates an elution of a therapeutic dose for up to about 35 days, while removing the hydrophilic polymer from that formulation increases the elution of drug at therapeutic levels to about 48 days (e.g. about 6 to 7 weeks). Removing the nonionic surfactant or including at a low amount (e.g. less than 5%(w/w)) from that formulation further increases the duration of elution of drug at therapeutic doses for up to 8 weeks. *See* Figures 12 and 13.

In other embodiments, the compositions comprise a sustained release formulation drug core comprising cyclosporine admixed with one ore more hydrophobic polymers and an optional nonionic surfactant to form a solid matrix composition, wherein the composition is in the form of a drug core and configured for placement within a lacrimal canaliculus. The solid matrix formulation and drug cores further comprise an impermeable sheath disposed at least partially over the solid matrix. The formulations were herein designed to topically deliver to the eye a daily therapeutic dose of cyclosporine for use in the treatment of dry eye.

Cyclosporine is an FDA approved drug, originally isolated from a fungus, indicated for the treatment of signs and symptoms of dry eye, a syndrome called keratoconjunctivitis sicca. Cyclosporine is an immunosuppressive drug and reduces inflammation including reducing activity of T cells in the conjunctiva tissue of the eye.

### Definitions

As used herein, the terms "a" or "an" are used, as is common in patent documents, to include one or more than one, independent of any other instances or usages of "at least one" or "one or more."

As used herein, the term "or" is used to refer to a nonexclusive or, such that "A or B" includes "A but not B," "B but not A," and "A and B," unless otherwise indicated.

As used herein, the term "about" is used to refer to an amount that is approximately, nearly, almost, or in the vicinity of being equal to or is equal to a stated amount, e.g., the state amount plus/minus about 5%, about 4%, about 3%, about 2% or about 1%.

As used herein, an "axis" refers to a general direction along which a member extends. According to this definition, the member is not required to be entirely or partially symmetric with respect to the axis or to be straight along the direction of the axis. Thus, in the context of this definition, any member disclosed in the present application characterized by an axis is not limited to a symmetric or a straight structure.

In this document, the term "proximal" refers to a location relatively closer to the cornea of an eye, and the term "distal" refers to a location relatively further from the cornea and inserted deeper into a lacrimal canaliculus.

In the appended claims, the terms "including" and "in which" are used as the plain-English equivalents of the respective terms "comprising" and "wherein." Also, in the following claims, the terms "including" and "comprising" are open-ended, that is, a system, assembly, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

### Compositions

In embodiments, the composition comprises the present sustained release formulation as a medical device, as a drug core, as a drug insert (e.g. present formulation and an outer layer or covering), and as a drug delivery system (e.g. drug insert or core and a body or retention element to maintain the drug insert or core in a desired location). In embodiments, the medical device (e.g. drug core or drug insert) may be placed in the lacrimal canaliculus.

In embodiments, the medical device comprises a substantially cylindrical diameter over the length of the medical device and may be configured for placement in a lacrimal canaliculus (e.g. intracanalicular plug), which may be in the shape of a ring or linear. In alternative embodiments, the drug insert is adapted to be placed in a body of the drug delivery system. The ocular drug delivery system, disclosed in more detail below, uses a body that is interchangeable with a drug insert and/or drug core comprising different drugs and/or different matrix to provide topical sustained release of the drug.

In embodiments, the lacrimal implant of the invention is configured as a sustained release device, releasing the incorporated ophthalmic drug (i.e, cyclosporine) in a therapeutically effective manner, e.g., at a rate that provides a therapeutically effective dosage for at least about 1 week, 2 weeks, 3 weeks, 4 weeks, 5 weeks, 6 weeks, 7 weeks, 8, weeks, 9 weeks 10 weeks, 11 weeks, or at least about 12 weeks or more. For cyclosporine, a therapeutic level is an average daily elution rate of at least 1.5µg/day of the drug. In an exemplary embodiment, the lacrimal implant is configured to be retained by the puncta for the duration of the intended controlled release of the therapeutic agent. In various embodiments, the duration of the intended controlled release of the therapeutic agent is at least about 1 week, 2 weeks, 3 weeks, 4 weeks, 5 weeks, 6 weeks, 7 weeks, 8, weeks, 9 weeks 10 weeks, 11 weeks, or at least about 12 weeks or more. In various embodiments at least 95% of the implanted implants are retained for the duration of the intended controlled release of the therapeutic agent. In an exemplary embodiment, the implant is retained by the puncta for a length of time to show therapeutic efficacy.

In embodiments, the present solid matrix sustained release ophthalmic formulations comprise from about 20% to about 80% w/w, from about 30% to about 80% w/w, from about 40% to about 80% w/w, from about 50% to about 80% w/w, from about 60% to about 80% w/w, or from about 70% to about 80% w/w of the ophthalmic drug. The ophthalmic drug is cyclosporine. The present solid matrix sustained release ophthalmic formulations comprise from 70% to 80% w/w of cyclosporine.

In certain embodiments, the present solid matrix sustained release ophthalmic formulations comprise about 75% w/w of cyclosporine.

In certain other embodiments, the present solid matrix sustained release ophthalmic formulations comprise 70%, about 72.5%, about 75%, about 77.5%, 80%, w/w of the ophthalmic drug. The ophthalmic drug is cyclosporine.

The present solid matrix sustained release ophthalmic formulations comprise from 70% to 80% w/w of the ophthalmic drug. In embodiments, the ophthalmic drug is present in the solid matrix formulation at 70%, about 71%, about 72%, about 73%, about 74%, about 75%, about 76%, about 77%, about 78%, about 79%, or 80% (w/w). The % numbers are inclusive of 0.5% above and below each of the whole percentage numbers, providing a range for "about". For example, about 75% is inclusive of 74.5, 74.75, 75, 75.25, 75.50 and each value in between thereof.

The present solid matrix sustained release ophthalmic formulations further comprises one or more hydrophobic polymers and optionally a nonionic surfactant. The ophthalmic drug is cyclosporine.

In embodiments, the present sustained release ophthalmic formulations comprise about 60 to about 240 µg of cyclosporine.

In embodiments, the solid matrix sustained release ophthalmic formulation for topical delivery of the ophthalmic drugs disclosed above is for use in the treatment of dry eye. In embodiments, a therapeutic dose of cyclosporine, as eluted from the present sustained release formulation when placed in or around the eye, is about 1.5 µg to about 3µg of cyclosporine a day.

The formulation may be prepared by dissolving the drug, polymer mixture and optional nonionic surfactant and then forming into a desired shape. The formulation may be extruded into a sheath body to form a drug insert, which may be used with a lacrimal implant or device (e.g. drug delivery system).

### Sustained Release Formulation Components

The present sustained release ophthalmic formulations comprise one or more hydrophobic polymers. The term "hydrophobic" as used herein is generally understood to be a polymer that has a limited affinity for water and does not mix well with water. For example, hydrophobic polymers may be non-polar and will aggregate in an aqueous solution and exclude water molecules. The exclusion of water maximizes the hydrogen bonding of the hydrophobic polymer, either to other hydrophobic polymers, a hydrophilic polymer or possibly even a surfactant. The hydrophobic polymers at least include polycaprolactone and polyvinyl acetate. In embodiments, hydrophobic polymers may further include for example, non-polar polymers, polyester polymers, PLGA, PLA, and polyanhydrides with hydrophobic co-monomer (e.g. carboxyphenoxypropane). In certain embodiments, the hydrophobic polymer may further include poly(D,L-lactic-co-glycolic acid)(PLGA), poly lactic acid (PLA), polyurethane, poly glycolic acid (PGA) or a combination thereof. In certain embodiments, the hydrophobic polymer may further comprise polyurethane, polyester, styrene, acrylonitrile, maleic anhydride, polyamide, polyimide, polydiene, poly(ethylene terephthalate) (PET), polyethylene, polypropylene, polyether, poly(fluorocarbon) polymers, poly(vinyl acetal), poly(vinyl chloride), poly(vinyl alcohol) (PVA), poly(vinyl ether), poly(vinyl ketone), poly(vinylpyrrolidone (PVP), poly(vinylpyridine), co-polymers thereof, or combinations thereof. The present solid matrix sustained release ophthalmic formulations comprise polycaprolactone and polyvinyl acetate as a first and second hydrophobic polymer of the one or more hydrophobic polymers.

The present solid matrix sustained release ophthalmic formulations comprise from 5% to 30% w/w of polycaprolactone. In other exemplary embodiments, the present solid matrix sustained release ophthalmic formulations comprise from about 15% to 30% w/w of polycaprolactone.

The present solid matrix sustained release ophthalmic formulations comprise from 0% to 20% w/w of polyvinyl acetate. In other exemplary embodiments, the present solid matrix sustained release ophthalmic formulations comprise from about 5% to 20% w/w of polyvinyl acetate.

In embodiments, the present solid matrix sustained release ophthalmic formulations comprise from about 10 to about 50% (w/w), from about 10 to about 45% (w/w), from about 10 to about 40% (w/w), from about 10 to about 35% (w/w), from about 10% to about 30% w/w, from about 10% to about 25% w/w, from about 10% to about 20% w/w, or from about 10 to about 15% (w/w) of the one or more hydrophobic polymers. In certain embodiments, the combined total of one or more hydrophobic polymers are not more than 30% w/w of the total formulation.

In certain embodiments, the present solid matrix sustained release ophthalmic formulations comprise from about 10% to about 35% w/w of one or more hydrophobic polymers. In embodiments, the one or more hydrophobic polymers are present in the solid matrix formulation at about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34% or about 35% (w/w) of the total hydrophobic polymer. The % numbers are inclusive of 0.5% above and below each of the whole percentage numbers, providing a range for "about". For example, about 20% is inclusive of 19.5, 19.75, 20, 20.25, 20.50 and each value in between thereof. The present solid matrix sustained release ophthalmic formulations comprise cyclosporine. The one or more hydrophobic polymers comprise polycaprolactone and polyvinyl acetate.

The present solid matrix sustained release ophthalmic formulations do not comprise silicone. The present solid matrix sustained release ophthalmic formulations do not comprise methacrylate polymers or monomers.

In embodiments, the present solid matrix sustained release ophthalmic formulations do not comprise a hydrophilic polymer. As used herein, the term "hydrophilic" is understood to be a polymer that has a strong affinity for water and may be readily soluble in water. For example, hydrophilic polymers may be polar and their interaction with water (and other polar) substances are more thermodynamically favorable than interactions with hydrophobic polymers or substances. In embodiments, hydrophilic polymers excluded from the present solid matrix sustained release ophthalmic formulations include for example, polar polymers, polysaccharides including alginate and chitosan, hydrophilic polyanhydrides, polyethylene glycol (PEG), proteins, DNA, and polyvinyl alcohol. In certain embodiments, the excluded hydrophilic polymers include polyethylene glycol (PEG) polymers, acrylate-derivatized PEG (PEGDA) polymers, polysaccharide polymers, hydrophilic polyanhydrides or a combination thereof. In certain embodiments, the excluded hydrophilic polymers include polyethylenimine (PEI), poly(ethylene glycol) (PEG), poly(oxyethylene), poly(ethylene oxide) (PEO), poly(acrylic acid) (PAA), poly(methacrylic acid), poly(vinyl alcohol) (PVA), poly(vinylpyrrolidone (PVP), polyelectrolytes, poly(maleic anhydride acid), poly(ether), acrylate-derivatized PEG (PEGDA) polymers, polysaccharide polymers, hydrophilic polyanhydrides, co-polymers thereof, or combinations thereof. In certain embodiments, the present solid matrix sustained release ophthalmic formulations do not comprise polyethylene glycol (PEG) polymers, acrylate-derivatized PEG (PEGDA) polymers, polysaccharide polymers, hydrophilic polyanhydrides or a combination thereof.

In embodiments, the present solid matrix sustained release ophthalmic formulations comprise a nonionic surfactant. In alternative embodiments, the present solid matrix sustained release ophthalmic formulations do not comprise a nonionic surfactant. As used herein "surfactant" refers to a compound that lowers the surface tension between two liquids or between a liquid and a solid. Surfactants are typically amphiphilic, meaning they comprise both a hydrophilic moiety and a hydrophobic moiety, such as fatty alcohol groups and compounds that form micelles in an aqueous solution. Nonionic surfactants have covalently bonded oxygen-containing hydrophilic groups, which are bonded to hydrophobic parent structures; an amphiphilic compound. The water-solubility of the oxygen groups is the result of hydrogen bonding. The differences between the individual types of nonionic surfactants are slight, and the choice is primarily governed based on the cost of special properties, e.g., effectiveness and efficiency, toxicity, dermatological compatibility and biodegradability, or permission for use in pharmaceutical products. In the instant solid matrix sustained release ophthalmic formulations, the choice of an individual surfactant may also be governed by improved efficiency in manufacturing, e.g. extrusion of the formulation into a mold or tubing, such as a sheath body. For example, use of tyloxapol or polysorbate may provide little difference in daily elution rate, however one may provide for improved extrusion during manufacturing depending on the choice of hydrophobic polymers and their overall % w/w in the matrix. In certain exemplary embodiments, the present solid matrix sustained release ophthalmic formulations comprise a polysorbate surfactant such as polysorbate 80.

In exemplary embodiments, the present solid matrix sustained release ophthalmic formulations do not comprise hydrophilic polymers or amphiphilic polymers or molecules.

Examples of nonionic surfactants include fatty alcohol ethoxylates, alkylphenol ethoxylates, fatty acid ethoxylates (e.g. polysorbate), certain ethoxylated fatty esters and oils, ethoxylated amines and/or fatty acid amides, terminally blocked ethoxylates, fatty acid esters of polyhydroxy compounds, fatty acid esters of glycerol, fatty acid esters of sorbitol (e.g. Spans), fatty acid esters of sucrose, alkyl polyglucosides, amine oxides, sulfoxides, polymers of alkyl aryl polyether alcohol (e.g. tyloxapol), polyoxyethylene ethers (e.g. BRIJ compounds) and phosphine oxides.

Polysorbate surfactants are ethoxylated sorbitan esters and include polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), and polysorbate 80 (polyoxyethylene (20) sorbitan monooleate), wherein the number 20 following the 'polyoxyethylene' part refers to the total number of oxyethylene -(CH₂CH₂O)-groups found in the molecule. The number following the 'polysorbate' part is related to the type of fatty acid associated with the polyoxyethylene sorbitan part of the molecule. Monolaurate is indicated by 20, monopalmitate is indicated by 40, monostearate by 60, and monooleate by 80. In exemplary embodiments, the present solid matrix sustained release ophthalmic formulations comprise the nonionic surfactant polysorbate 80.

BRIJ nonionic surfactants are polyoxyethylene ethers and include, polyoxyethylene (20) oleyl ether, polyoxyethylene (10) oleyl ether, polyoxyethylene (2) oleyl ether, polyoxyethylene (100) stearyl ether, polyoxyethylene (20) cetyl ether, polyoxyethylene (10) cetyl ether, polyoxyethylene (10) stearyl ether, polyoxyethylene (4) lauryl ether, polyoxyethylene (20) stearyl ether, polyoxyethylene (2) cetyl ether, and polyoxyethylene (2) stearyl ether.

Span nonionic surfactants are sorbitan esters that include sorbitan oleate, sorbitan stearate, sorbitan laurate, sorbitane trioleate, sorbitan tristearate, sorbitan sesquioleate, and sorbitan monopalmitate. In embodiments, the present solid matrix sustained release ophthalmic formulations comprise the nonionic surfactant sorbitan ester. In certain embodiments, the present solid matrix sustained release ophthalmic formulations comprise a combination of the nonionic surfactants sorbitan ester (e.g. Span 40) and polysorbate (e.g. polysorbate 80). In certain embodiments, the present solid matrix sustained release ophthalmic formulations comprise a combination of the nonionic surfactants sorbitan ester (e.g. Span 40) and polysorbate (e.g. polysorbate 80), wherein the solid matrix does not comprise a hydrophilic polymer as disclosed above.

In certain embodiments, the present solid matrix sustained release ophthalmic formulations comprise from about 1% to about 10% w/w of a nonionic surfactant. In embodiments, the nonionic surfactant is present in the solid matrix formulation at about 1%, about 2%, about 3%, about 4%, about 5%, about 6%, about 7%, about 8%, about 9% or about 10% (w/w). The % numbers are inclusive of 0.5% above and below each of the whole percentage numbers, providing a range for "about". For example, about 4% is inclusive of 3.5, 3.75, 4, 4.25, 4.50 and each value in between thereof. In certain embodiments, the present solid matrix sustained release ophthalmic formulations further comprise polycaprolactone. In exemplary embodiments, the nonionic surfactant is a polysorbate.

In certain embodiments, the present solid matrix sustained release ophthalmic formulations comprise from about 0% to about 25% w/w of a nonionic surfactant. In embodiments, the nonionic surfactant is present in the solid matrix formulation at about 0%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, or about 25% (w/w). The % numbers are inclusive of 0.5% above and below each of the whole percentage numbers, providing a range for "about". For example, about 20% is inclusive of 19.5, 19.75, 20, 20.25, 20.50 and each value in between thereof. In certain embodiments, the present solid matrix sustained release ophthalmic formulations further comprise polycaprolactone. In exemplary embodiments, the nonionic surfactant is a polysorbate.

In certain embodiments, the present solid matrix sustained release ophthalmic formulations comprise a nonionic surfactant selected from tyloxapol, sorbitan esters, polyoxyethylene ethers, a polysorbate or a combination thereof.

In embodiments, the present sustained release ophthalmic formulations comprise a) one or more hydrophobic polymers; b) an optional nonionic surfactant; and, c) the ophthalmic drug, wherein the formulation does not comprise a hydrophilic polymer and wherein the hydrophobic polymer is polycaprolactone and is present from 5% (w/w), the nonionic surfactant is polysorbate 80 and is present from about 0 to 22.5% (w/w), and the ophthalmic drug is cyclosporine and is present up to 80% (w/w).

In embodiments, the present solid matrix sustained release ophthalmic formulations comprise one or more hydrophobic polymers; an optional nonionic surfactant and ophthalmic drug, wherein the hydrophobic polymer is polycaprolactone and is present from 5 to 30% (w/w), the nonionic surfactant is polysorbate 80 and is present from 0 to 10% (w/w), and the ophthalmic drug is cyclosporine and is present from 70 to 80% (w/w).

In embodiments, the present solid matrix sustained release ophthalmic formulations comprise a) one or more hydrophobic polymers; and, b) the ophthalmic drug, wherein the formulation does not comprise a hydrophilic polymer or a nonionic surfactant, wherein a first hydrophobic polymer is polycaprolactone and is present from 15 to 30% (w/w), a second hydrophobic polymer is polyvinyl acetate and is present from 0 to 15% (w/w), and the ophthalmic drug is cyclosporine and is present from 70 to 80% (w/w).

In embodiments, the present solid matrix sustained release ophthalmic formulations comprise a) one or more hydrophobic polymers; b) an optional nonionic surfactant; and, c) the ophthalmic drug, wherein the formulation does not comprise a hydrophilic polymer, wherein a first hydrophobic polymer is polycaprolactone and is present from 5 to 30% (w/w), a second hydrophobic polymer is polyvinyl acetate and is present from 5 to 20% (w/w), the nonionic surfactant is polysorbate 80 and is present from 0 to 5% w/w, and the ophthalmic drug is cyclosporine and is present from 70 to 80% (w/w).

In certain embodiments, an impermeable sheath body (disclosed in more detail below) is disposed over at least a portion of the solid matrix composition.

### Lacrimal Implants

In embodiments, provided herein are lacrimal implants comprising a punctal plug comprising a plug body and a drug insert, wherein the insert comprises; a drug core comprising any one of the present solid matrix sustained release ophthalmic formulations disclosed herein; and, an impermeable sheath body partially covering the drug core, wherein the sheath body is configured to provide an exposed proximal end of the drug core in direct contact with tear fluid that releases an ophthalmic drug to the eye when the drug insert is disposed within a channel of the punctal plug and the punctal plug is inserted into the lacrimal canaliculus of a patient.

In certain embodiments, the any one of the present solid matrix sustained release ophthalmic formulations disclosed herein are configured as a medical device for the delivery of the ophthalmic drug to the eye. Those medical devices may take the shape of a depot, a lacrimal implant with a separate body, an intracanalicular plug that does not further comprise a separate plug body or a sheath body.

In certain embodiments, the intracanalicular plug comprises a polymeric coating or layer completely or partially surrounding the plug. In embodiments, the medical device may comprise a coating or an internal filament to provide structural integrity to the medical device. In embodiments, the medical device has a substantially cylindrical shape wherein the diameter of the entire medical device is approximately the same at the time of placement in, on or near the eye.

In certain embodiments, the compositions of the invention comprise an implant including a distinct solid matrix formulation drug core or integrated drug or other agent disposed in at least one of the first member **305** or the second member **310** of the implant body, to provide a sustained release of the therapeutic agent (used interchangeably herein with ophthalmic drug). For instance, the drug core or integrated drug or other agent disposed may be disposed in the cavity **458** of the lacrimal implant **400** to provide a sustained drug or other therapeutic agent release.

An exemplary implant of use in the methods of the invention is configured to deliver a therapeutic agent to one or more of an eye.

The drug is delivered systemically to the subject through the eye. A therapeutic agent core can comprise one or more therapeutic agents, and in some examples, one or more matrix materials to provide sustained release of the drug or other agents.

The drug core (used interchangeably herein with the present solid matrix sustained release ophthalmic formulation) is inserted into cavity **458.**

The compositions comprise a drug insert comprising a sheath body and a present sustained release ophthalmic formulation. The sheath body can comprise appropriate shapes and materials to control the migration of ophthalmic drug from the drug core. In some embodiments, the sheath body houses the drug core and can fit snugly against the core. The sheath body is made from a material that is substantially impermeable to the anti-inflammatory agent so that the rate of migration of the agent may be largely controlled by the exposed surface area of the drug core that is not covered by the sheath body. In many embodiments, migration of the ophthalmic drug through the sheath body can be about one tenth of the migration of ophthalmic drug through the exposed surface of the drug core, or less, often being one hundredth or less. In other words, the migration of the ophthalmic drug through the sheath body is at least about an order of magnitude less that the migration of anti-inflammatory agent through the exposed surface of the drug core. Suitable sheath body materials include polyimide, polyethylene terephthalate (hereinafter "PET"). The sheath body has a thickness, as defined from the sheath surface adjacent the core to the opposing sheath surface away from the core, from about 0.00635 mm to about 0.0381 mm (from about 0.00025" to about 0.0015"). The total diameter of the sheath that extends across the core ranges from about 0.2 mm to about 1.2 mm. In embodiments, the drug core has a diameter from about 0.55 to about 0.70mm. In certain embodiments, the drug core has a diameter of about 0.61mm. The core may be formed by dip coating the core in the sheath material. Alternatively, or in combination, the sheath body can comprise a tube and the core introduced into the sheath, for example as a liquid or solid that can be slid, injected or extruded into the sheath body tube. The sheath body can also be dip coated around the core, for example dip coated around a pre-formed core.

It is generally understood that when the present solid matrix formulation is at least partially surrounded by a sheath body, the hydrophobic polymers do not erode. In other words, they are not biodegradable via hydrolysis or oxidation, even when those polymers may be biodegradable under different conditions (e.g., when not protected by a sheath body). Hence, while hydrophilic moieties present in the polymers and/or surfactants of the present solid matrix sustained release ophthalmic formulations may bind water molecules, such as present in tear fluid, the polymers do not generally undergo hydrolysis during the treatment period.

The sheath body can be provided with additional features to facilitate clinical use of the implant. For example, the sheath may receive a drug core that is exchangeable while the implant body, retention structure and sheath body remain implanted in the subject. The sheath body is often rigidly attached to the retention structure as described above, and the core is exchangeable while the retention structure retains the sheath body. In specific embodiments, the sheath body can be provided with external protrusions that apply force to the sheath body when squeezed and eject the core from the sheath body. Another drug core can then be positioned in the sheath body. In many embodiments, the sheath body or retention structure may have a distinguishing feature, for example a distinguishing color, to show placement such that the placement of the sheath body or retention structure in the canaliculus or other body tissue structure can be readily detected by the subject. The retention element or sheath body may comprise at least one mark to indicate the depth of placement in the canaliculus such that the retention element or sheath body can be positioned to a desired depth in the canaliculus based on the at least one mark.

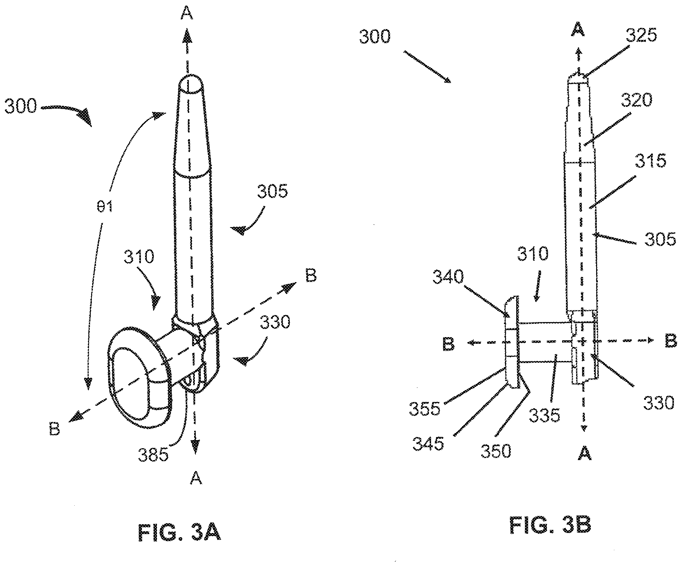

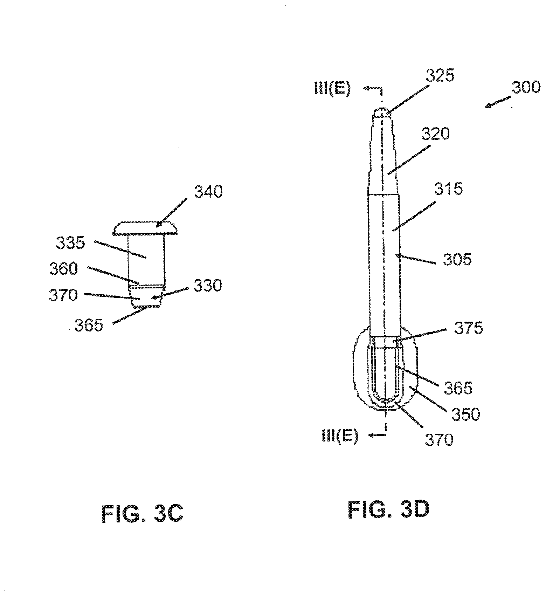

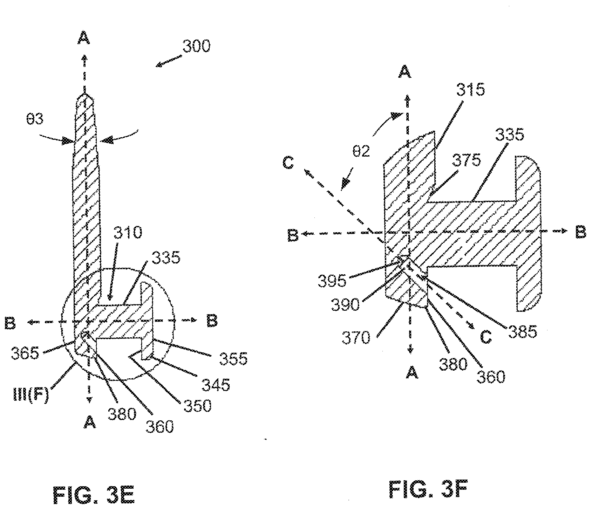

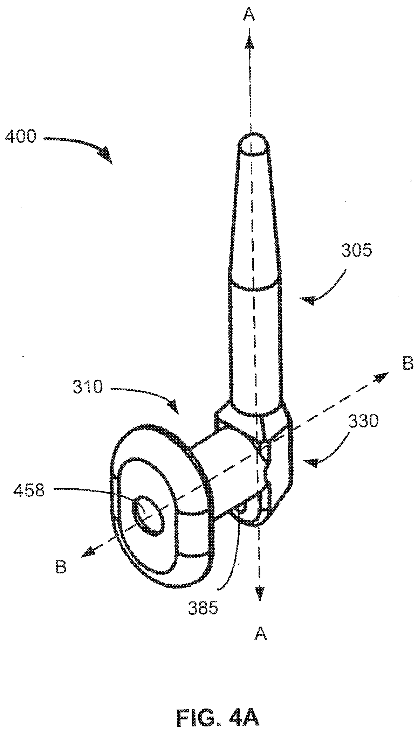

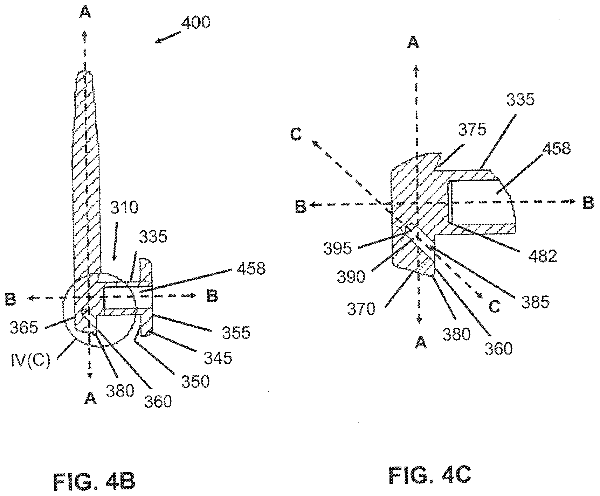

FIGS. 3-6 illustrate exemplary embodiments of lacrimal implants of use with the present formulations and in methods of the invention. The exemplary implants are insertable through a lacrimal punctum **212, 214** and into its associated canaliculus **208, 210.** Exemplary lacrimal implants of use in the present invention comprise a first member, a second member and a heel, such as the first member **305,** the second member **310** and the third member or heel **330** depicted in FIG. 3A. Exemplary lacrimal implants further comprise a bore that is formed in the heel, for example, the bore **385** formed in the third member or heel **330** in FIG. 3A. In some embodiments, exemplary lacrimal implants further comprise a cavity **458** (e.g., lacrimal implants illustrated in FIG. 4A).

Referring to FIG. 3A, where a perspective view of an exemplary lacrimal implant **300** of use in the present methods is depicted, the first member **305** is characterized by a first axis A and the second member **310** is characterized by a second axis B.

The third member or heel **330** is configured to connect the first member **305** and the second member **310** at a first angle θ₁, where θ₁ is defined by the first axis A with respect to the second axis B. For instance, in FIG. 3A, the first angle θ₁ refers to the angle originating at the first axis A and turning counterclockwise from the first axis A to the second axis B. In some embodiments, the first axis A and the second axis B are in the same plane and intersect each other. In some embodiments, the first axis A is in a plane other than the plane of the second axis B, and the first axis A and the second axis B do not intersect. In such embodiments, the first angle θ₁ refers to the angle defined by a parallel line of the first axis A with respect to the second axis B. This parallel line of the first axis A lies in the same plane as the second axis and intersects with the second axis.

In some embodiments, the first angle θ₁ is from about 30 degrees to about 150 degrees, from about 45 degrees to about 135 degrees, or from about 75 degrees to about 105 degrees. For example, in some embodiments, the first angle θ₁ is approximately 90 degrees.

In some embodiments, the overall dimension of the implant along the first axis is from about 4 mm to about 8 mm. In an exemplary embodiment, the overall dimension along the first axis is about 5 mm to about 7 mm. In various embodiments, the overall dimension along the first axis is about 6.3 mm.

In various embodiments, the overall dimension along the second axis B is from about 1 mm to about 3 mm, e.g., from about 1.2 mm to about 1.9 mm.

In some embodiments, the overall dimension along the first axis is approximately 6.3 mm and the overall dimension along the second axis is approximately 1.2 mm. In various embodiments, the overall dimension along the first axis is approximately 6.3 mm and the overall dimension along the second axis is approximately 1.9 mm. In some embodiments, the overall dimension along the first axis is approximately 4.8 mm and the overall dimension along the second axis is approximately 1.9 mm.

In some embodiments, the first member **305** is configured to extend into a canaliculus, while the second member **310** is configured to reside in the vertical portion **220, 222** of the canaliculus and to extend to the opening of, or out of the opening of, the associated puncta. When a lacrimal implant **300** of such configuration is inserted into a canaliculus, the intersection of the first axis A and the second axis B resides generally at a curvature of the canaliculus, such as the canaliculus curvature **250** in FIG. 2. In some embodiments, the first member **305** and the second member **310** are connected at the first angle, and that angle is at least about 45 degree, thereby forming an angled intersection between the first member and the second member. In various embodiments, when the lacrimal implant **300** is positioned in the lacrimal canaliculus, at least a portion of the angled intersection is biased against a canaliculus curvature of the lacrimal canaliculus. In this embodiment, the lacrimal implant **300** uses anatomical structures to facilitate the retention of the implanted lacrimal implant **300.**

FIG. 3B depicts a side view of an exemplary lacrimal implant **300** of the invention. In some embodiments, the first member **305** includes an intermediate segment **315,** a tip segment or tip **325,** and a forward segment **320** in between the forward segment and tip segment. While the intermediate segment **315** is configured to be connected to the second member **310** by the third member or heel **330,** the tip segment or tip **325** is configured to be inserted through a punctum prior to the other two segments of the first member **305** and prior to the other members of the lacrimal implant **300.**

In some embodiments, the intermediate segment **315,** the forward segment **320** and the tip segment or tip **325** are distinguishable from each other in general by their shapes. For example, in some embodiments, the intermediate segment **315** has a generally cylindrical shape with a diameter that is larger than the diameter of the tip segment or tip **325.** In various embodiments, the forward segment **320** is tapered and has a conical shape, such that the forward segment **320** connects the intermediate segment **315** at one end and the tip segment or tip **325** at the other end. In some embodiments, the transition from the intermediate segment **315** to the forward segment **320** or the transition from the forward segment **320** to the tip segment or tip **325** is gradual and smooth such that no distinguishable edge exists at the transition.

In some embodiments, the intermediate segment **315** has a cylindrical shape. In various embodiments, the intermediate segment has a circular cross section, an elliptic cross section, or a polygonal cross section. The intermediate segment **315** is of any useful combination of length and diameter.

In some embodiments, the intermediate segment **315** has a diameter that is from about 0.4 mm to about 0.8 mm. For example, in some embodiments the diameter of the intermediate segment **315** is from about 0.53 mm to about 0.63 mm. In some embodiments, the intermediate segment **315** has a length along the first axis A that is from about 0.5 mm to about 3.5 mm. For example, in some embodiments the length of the intermediate segment **315** is from about 1 mm to about 2.8 mm.

In some embodiments, the tip segment or tip **325** is substantially a semi-sphere, or a portion of a semi-sphere. In exemplary embodiments, the semi-sphere, or portion therapy, has a radius that is from about 0.05 mm to about 0.3 mm. For example, in some embodiments, the radius of the tip segment or tip **325** is approximately 0.20 mm.

In some embodiments, the forward segment **320** has a conical configuration, tapering from the diameter of the intermediate segment **315** as it approaches the tip segment or tip **325.** In some embodiments, the forward segment **320** is short and is tapered steeply, thus forming a wider taper angle. The forward segment **320** can also be long and tapered more gradually, thus forming a narrower taper angle. The tapering angle θ₃ is illustrated in FIG. 3E. In some embodiments, the tapering angle θ₃ is from about 2° to about 10°. For example, in some embodiments the tapering angle θ₃ is from about 3.8° to about 7.8°. In some embodiments, θ₃ is about 7.8°. In some embodiments, the forward segment **320** has a length along the first axis A that is from about 1 mm to about 5 mm. For example, in some embodiments the length of forward segment **320** is from about 1.7 mm to about 3.5 mm.

Referring to FIG. 3B, in some embodiments of implants of use in the present method, the second member **310** includes an upright segment **335** that extends from the third member or heel **330** generally along the direction of the second axis B. In various embodiments, the second member **310** further includes a head segment **340** that attaches to the upright segment **335** at an end opposite to the third member or heel **330.** In some embodiments, the second member **310** is configured such that the upright segment **335** resides in the vertical portion of the canaliculus while the head segment **340** contacts the tissue surrounding the exterior of the punctum when the lacrimal implant **300** is positioned in the lacrimal canaliculus. In an exemplary embodiment, illustrated in FIGS. 3A-3F, the upright segment **335** has a cylindrical shape and the head segment **340** has an oval or oblong configuration. However, it will be appreciated that any other suitable shapes or configurations can be used and are within the scope of the present invention. For example, in various embodiments, the upright segment **335** is configured to be a conical; the head segment **340** is configured to have a circular, elliptical or polygonal cross section.

In some embodiments, the upright segment **335** has a characteristic diameter that is from about 0.7 mm to about 0.9 mm. For example, in some embodiments, the characteristic diameter of the upright segment **335** is about 0.8 mm.

In some embodiments, the upright segment **335** has a length in the direction of the second axis B that is from about 0.7 mm to about 1.5 mm. For example, in some embodiments the length of upright segment **335** along the direction of the second axis B is about 0.9 mm.

Generally, the head segment **340** has a cross section characterized by a minor axis and a major axis. The minor axis and the major axis refer to the shortest characteristic diameter and the longest characteristic diameter of the cross section, respectively. As such, the minor axis is equal to or less than the major axis. For instance, in some embodiments where the head segment **340** has a circular cross section, the minor axis and the major axis are of equal length. In various embodiments, the head segment **340** has an oval or oblong cross section, and the minor axis is shorter than the major axis. In some embodiments, the head segment **340** is elongated in a direction that is parallel to the first axis A. The major axis indicates the extension of the first member **305** and facilitates positioning of the lacrimal implant **300** in the punctum and canaliculus. In some embodiments, the major axis is from about 1.5 mm to about 2.5 mm. In various embodiments, the minor axis is from about 1 mm to about 1.5 mm. For example, in some embodiments, the major axis and the minor axis head segment **340** are approximately 1.9 mm and 1.3 mm respectively. In some embodiments, the head segment **340** has a thickness in the direction of the second axis that is from about 0.2 mm to about 0.4 mm. For example, in some embodiments, the thickness of the head segment **340** in the direction of the second axis is approximately 0.3 mm.

Referring still to FIG. 3B, exemplary head segment **340** comprises an under-surface **350** facing towards the third member or heel **330** and an outer-surface **355** that faces away from the third member or heel **330.** Exemplary head segment **340** further comprises an edge surface **345** that couples the under-surface **350** and the outer-surface **355.** The distance between the under-surface **350** and the outer-surface **355** can be readily varied. In some embodiments, the distance is from about 0.2 mm to about 0.4 mm.

In some embodiments, the outer-surface **355** is smaller than the under-surface **350** and is substantially flat. In various embodiments, the edge surface **345** is tapered, curved, angular, or multifaceted. In some embodiments, the edge surface **345** has a radius of curvature that is from about 0.2 mm to about 0.7 mm. In some embodiments, the under-surface **350** is in general flat and is configured to contact the exterior tissue surrounding the punctum when the lacrimal implant **300** is positioned in the lacrimal canaliculus.

In some embodiments, the third member or heel **330** includes an upper surface **360** a lower surface **365** and side surfaces **370.** In the illustrated embodiments, the bore **385** extends from the upper surface **360** into the third member or heel **330.** In some embodiments, the upper surface **360** and the lower surface **365** are substantially flat and separated from each other by a distance. Such distance is readily variable and is typically about 0.3 mm to about 0.7 mm. For instance, in some embodiments, the upper surface **360** and the lower surface **365** are separated by a distance that is from about 0.4 mm to 0.6 mm (e.g., about 0.53 mm). In some embodiments, the upper surface **360** extends beyond the intersection with the second member **310.** In some embodiments, the upper surface **360** extends beyond the intersection with the second member **310** for a distance that is from about 0.3 to about 0.6 mm. The upper surface **360** can also be joined with the side surfaces **370.** In various embodiments, upper surface **360** and side surfaces 370 are joined by a curved intersection **380.** In some embodiments, the curved intersection **380** has a radius of curvature that is from about 0.04 mm to about 0.08 mm.

Referring now to FIGS. 3D and 3F, in some embodiments, the third member or heel **330** includes a heel connecting segment **375** configured to couple the third member or heel **330** to the first member **305** or to the intermediate segment **315** of the first member **305.** The heel connecting segment **375** is of readily variable shape, including flat or curved structures. In FIG. 3F, a width of the heel connecting segment **375** in the direction of the second axis B varies along the direction of the first axis A. For example, the heel connecting segment **375** has a smaller width at or near the side surfaces **370** than the diameter of the intermediate segment **315** of the first member **305.** In some embodiments, at or near the intersection with the intermediate segment **315,** the heel connecting segment **375** increases the width and thus forms a notch as depicted in FIG. 3F. It will be appreciated that the notch can be either deeper or shallower along both the first axis A and the second axis B before it meets the first member **305** or the second member **310.**

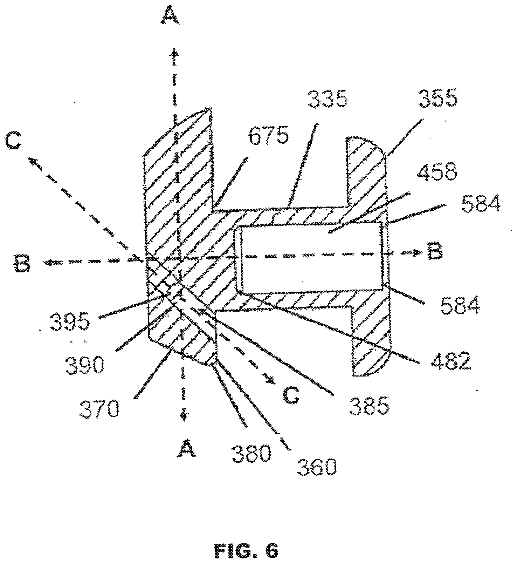

A notch is not a required feature in the implants of the present invention. In some embodiments, the heel connecting segment **375** has the same dimension as the diameter of the intermediate segment **315.** For example, the thickness of the third member or heel **330** along the second axis B is equal to the diameter of the intermediate segment **315** of the first member **305.** For example, in some embodiments, both the thickness of the third member or heel **330** in the direction of the second axis B and the diameter of the intermediate segment **315** are from about 0.53 mm to about 0.63 mm. In such configurations, the third member or heel **330** couples with the intermediate segment **315** without forming a notch, as illustrated by the alternative heel connecting segment **675** in FIG. 6.

By way of illustration, the third member or heel **330** depicted in FIGS. 3A-3F is substantially parallel to the first axis A of the first member **305.** It would be appreciated that this is unnecessary. In some embodiments, the third member or heel **330** can form an angle with relation to the first axis A.

Exemplary structures of the bore **385** are detailed in FIGS. 3E and 3F, where a cross sectional view and a partial enlarged cross-sectional view of the lacrimal implant **300** are provided. The bore **385** is configured to receive a tip or other protrusion of an external insertion tool for facilitating insertion of the lacrimal implant **300** into a lacrimal punctum. The configuration, including size, shape, angle (θ₂) and position of the bore in the heel are readily adjustable to facilitate the mating of the insertion tool with the bore, the flexibility of the heel, or the retention of the lacrimal implants. Depending on the purpose or use of the implant and the materials used for making the heel, the characteristics of the bore noted above are readily varied. Configurations of the bore **385** disclosed herein are illustrative and any other suitable configurations are within the scope of the present invention.

In FIG. 3F, an exemplary bore **385** is characterized by a third axis C and a second angle θ₂ that is defined by the first axis with respect to the third axis A in a similar way as the first angle θ₁. In some embodiments, the second angle θ₂ is from about 15° to about 90°. For example, in some embodiments, the second angle θ₂ is about 45°.

In some embodiments, the bore **385** has a depth along the direction of the third axis C that is from about 0.3 mm to about 0.7 mm. For example, in some embodiments the depth of the bore **385** is approximately 0.4 mm and in some embodiments is approximately 0.6 mm. The bore **385** may include a bore shaft **390** that is generally cylindrical, with a circular, elliptical, oval, or polygonal cross section. The bore **385** may further include a bore tip **395** at which the bore shaft **390** terminates. An exemplary bore tip **395** generally has a semispherical configuration. In some embodiments, the bore shaft **390** has a characteristic diameter that is from about 0.1 mm to about 0.3 mm. In some embodiments, the characteristic diameter of the bore is approximately 0.17 mm. As will be appreciated, the shapes, sizes, orientations disclosed in the present application are illustrative, and any other suitable shapes, sizes, or orientations are within the scope of the present application. In addition, it will be appreciated that the opening of the bore can be positioned closer to the second member or closer to the edge of the heel.

FIG. 4A-4C illustrates an exemplary lacrimal implant **400** that is insertable through a lacrimal punctum **212, 214** and into its associated canaliculus **208, 210.** In FIG. 4A, the lacrimal implant **400** comprises a cavity **458** that is configured to house a therapeutic agent core or other materials for release into an eye or surrounding tissues for treatment of various ocular, sinus or other diseases.

In the illustrated exemplary embodiment, the cavity **458** is formed in the head segment **340** and has an opening through the outer-surface **355.** The cavity **458** can be shallow such that it stays within the head segment **340.** The cavity **458** can be also deeper and extend beyond the head segment **340** and into the upright segment **335.** Illustrated exemplary cavity **458** is in general substantially cylindrical with a circular cross section. Any other suitable configuration is within the scope of the present application. For example, in some embodiments, the cavity **458** has a truncated spherical configuration, or has a cylindrical configuration with an oblong or a polygonal cross section.

In some embodiments, the cavity **458** has a depth in the direction of the second axis B that is about from 0.2 mm to about 1.4 mm. For example, in some embodiments, the depth of the cavity **458** is approximately 1.2 mm. In some embodiments, the cavity **458** has a diameter that is from about 0.3 mm to about 0.7 mm. For example, in some embodiments the diameter of the cavity **458** is from about 0.42 mm to about 0.55 mm. In an exemplary embodiment, the cavity **458** extends into the upright segment **335,** and the diameter of the cavity **458** is smaller than the diameter of the upright segment **335.**

Referring to FIG. 4C, the cavity **458** includes a bottom **482.** In various embodiments, the bottom **482** is rounded. In various embodiments, the rounded bottom has a radius of curvature that is from about 0.03 mm to about 0.07 mm.

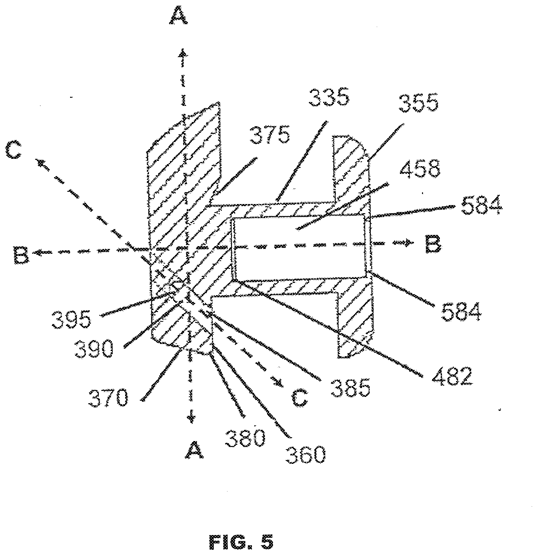

FIG. 5 depicts exemplary configurations of the cavity **458.** In FIG. 5, the cavity **458** includes a lip **584** or other retaining structure positioned at the opening of the cavity **458.** The lip **584** or the other retaining structure are optionally configured to partially enclose the cavity **458,** e.g, prevent a therapeutic agent core or other materials from moving out of the cavity **458.** In some embodiments, the lip **584** is a square cross-sectional annulus that extends down from the outer-surface **355** into the cavity **458** and extends inwardly towards the center of the opening of the cavity **458.** In some embodiments, the lip **584** is of a tab configuration and includes a plurality of spaced lips that extend inwardly into the opening of the cavity **458.** The lip **584** may extend downwardly from about 0.02 mm to about 0.1 mm and inwardly from about 0.02 mm to about 0.1 mm. For example, in some embodiments, the lip **584** extends about 0.05 mm downwardly or inwardly.

Exemplary lacrimal implants of use in methods of the present invention are made of various materials including plastic, rubber, polymer, or composite. Exemplary lacrimal implants of the present invention formed from one or more material including plastic, rubber, polymer, composites, or other appropriate materials. In some embodiments, the lacrimal implants are formed from liquid silicone rubber. For instance, in exemplary embodiments, lacrimal implants are formed from a material marketed as NuSil 4840 liquid silicone rubber, NuSil 4870, or a mixture including such a liquid silicone rubber. Examples of such a mixture include a material marketed as 6-4800, which comprises NuSil 4840 with from about 1% to about 5%, e.g., from about 2% to about 4% 6-4800.

In some embodiments, the lacrimal implant is formed from biodegradable materials, for instance, biodegradable elastic materials including cross-linked polymers, such as poly (vinyl alcohol). In some embodiments, the lacrimal implant can comprise a co-polymer, such as silicone/polyurethane co-polymer, silicone/urethane, silicone/poly (ethylene glycol) (PEG), and silicone/2hydroxyethyl methacrylate (HEMA). As discussed in commonly-owned Utkhede et al., U.S. patent application Ser. No. 12/231,986, entitled "DRUG CORES FOR SUSTAINED RELEASE OF THERAPEUTIC AGENTS," filed Sep. 5, 2008, urethane-based polymer and copolymer materials allow for a variety of processing methods and bond well to one another.

The hardness of the material is selected to facilitate or alter the retention of the lacrimal implant within the lacrimal punctum and its associated canaliculus. Accordingly, in some embodiments, a material having a durometer rating of from about 20D to about 80D, e.g., about 30D to about 70D, e.g., from about 40D to about 60D is of use to adjust parameters such as patient comfort and retention. For example, in some embodiments, the durometer rating of the material used to form the lacrimal implants is approximately 40D. Materials other than those exemplified above providing a durometer rating for the lacrimal implants within the stated ranges, and particularly that is about 40D are also of use. In some embodiments, a harder material or softer material is utilized for the entire lacrimal implant or for portions thereof. In such case, the lacrimal implants are formed from the materials that provide a durometer rating of about 70D.

In some embodiments, the lacrimal implants of use in the present methods are formed of multiple materials, where certain members or portions of the lacrimal implants are formed with materials having different properties. For example, in some embodiments the first member **305** is formed of a harder durometer rated material while the second member **310** is formed of a softer durometer rated material. In some embodiments, the first member **305** is formed of a softer durometer rated material while the second member **310** is formed of a harder durometer rated material. In some embodiments the third member or heel **330** is formed of a harder durometer rated material than one or more parts of the remainder of the second member **310.** In various embodiments, the third member or heel **330** is formed of a softer durometer rated material than the remainder of the second member **310.**

Exemplary implants of use in the invention can be formed by methods known in the art, including, but not limited to, machining a blank to the desired shape and size and molding the material forming the implant.

The implant can be one of any number of different designs that releases anti-inflammatory agents and or drugs for a sustained period of time. The disclosures of the following patent documents are relevant: U.S. Application Serial No. 60/871,864 (filed December 26, 2006 and entitled Nasolacrimal Drainage System Implants for Drug Therapy); U.S. Application Serial No. 11/695,537 (filed April 2, 2007 and entitled Drug Delivery Methods, Structures, and Compositions for Nasolacrimal System); U.S. U.S. Application Serial No. 12/332,219 (filed December 10, 2008 and entitled Drug Delivery Methods, Structures, and Compositions for Nasolacrimal System); U.S. Application Serial No. 60/787,775 (filed March 31, 2006 and entitled Nasolacrimal Drainage System Implants for Drug Therapy); U.S. Application Serial No. 11/695,545 (filed April 2, 2007 and entitled Nasolacrimal Drainage System Implants for Drug Therapy); U.S. Application Serial No. 60/585,287 (filed July 2, 2004 and entitled Treatment Medium Delivery Device and Methods for Delivery of Such Treatment Mediums to the Eye Using Such a Delivery Device); U.S. Application Serial No. 11/571,147 (filed December 21, 2006 and entitled Treatment Medium Delivery Device and Methods for Delivery of Such Treatment Mediums to the Eye Using Such a Delivery Device); U.S. Application Serial No. 60/970,696 (filed September 7, 2007 and entitled Expandable Nasolacrimal Drainage System Implants); U.S. Application Serial No. 60/974,367 (filed September 21, 2007 and entitled Expandable Nasolacrimal Drainage System Implants); U.S. Application Serial No. 60/970,699 (filed September 7, 2007 and entitled Manufacture of Drug Cores for Sustained Release of Therapeutic Agents); U.S. Application Serial No. 60/970,709 (filed September 7, 2007 and entitled Nasolacrimal Drainage System Implants for Drug Delivery); U.S. Application Serial No. 60/970,720 (filed September 7, 2007 and entitled Manufacture of Expandable Nasolacrimal Drainage System Implants); U.S. Application Serial No. 60/970,755 (filed September 7, 2007 and entitled Prostaglandin Analogues for Implant Devices and Methods); U.S. Application Serial No. 60/970,820 (filed September 7, 2007 and entitled Multiple Drug Delivery Systems and Combinations of Drugs with Punctal Implants); U.S. Application Serial No. 61/066,223 (filed February 18, 2008 and entitled Lacrimal Implants and Related Methods); U.S. Application Serial No. 61/049,347 (filed April 30, 2008 and entitled Lacrimal Implants and Related Methods); U.S. Application Serial No. 61/033,211 (filed March 3, 2008 and entitled Lacrimal Implants and Related Methods); U.S. Application Serial No. 61/049,360 (filed April 30, 2008 and entitled Lacrimal Implants and Related Methods); U.S. Application Serial No. 61/052,595 (filed May 12, 2008 and entitled Lacrimal Implants and Related Methods); U.S. Application Serial No. 61/075,309 (filed June 24, 2008 and entitled Lacrimal Implants and Related Methods); U.S. Application Serial No. 61/154,693 (filed February 23, 2009 and entitled Lacrimal Implants and Related Methods); U.S. Application Serial No. 61/209,036 (filed March 2, 2009 and entitled Lacrimal Implants and Related Methods); U.S. Application Serial No. 61/209,630 (filed March 9, 2009 and entitled Lacrimal Implants and Related Methods); U.S. Application Serial No. 61/036,816 (filed March 14, 2008 and entitled Lacrimal Implants and Related Methods); U.S. Application Serial No. 61/271,862 (filed July 27, 2009 and entitled Lacrimal Implants and Related Methods); U.S. Application Serial No. 61/252,057 (filed October 15, 2009 and entitled Lacrimal Implants and Related Methods); U.S. Application Serial No. 12/710,855 (filed February 23, 2010 and entitled Lacrimal Implants and Related Methods); U.S. Application Serial No. 60/871,867 (filed December 26, 2006 and entitled Drug Delivery Implants for Inhibition of Optical Defects); U.S. Application Serial No. 12/521,543 (filed December 31, 2009 and entitled Drug Delivery Implants for Inhibition of Optical Defects); U.S. Application Serial No. 61/052,068 (filed May 9, 2008 and entitled Sustained Release Delivery of Latanoprost to Treat Glaucoma); U.S. Application Serial No. 61/052,113 (filed May 9, 2008 and entitled Sustained Release Delivery of Latanoprost to Treat Glaucoma); U.S. Application Serial No. 61/108,777 (filed October 27, 2008 and entitled Sustained Release Delivery of Latanoprost to Treat Glaucoma); U.S. Application Serial No. 12/463,279 (filed May 8, 2009 and entitled Sustained Release Delivery of Active Agents to Treat Glaucoma and Ocular Hypertension); U.S. Application Serial No. 61/049,337 (filed April 30, 2008 and entitled Lacrimal Implants and Related Methods); U.S. Application Serial No. 12/432,553 (filed April 29, 2009 and entitled Composite Lacrimal Insert and Related Methods); U.S. Application Serial No. 61/049,317 (filed April 30, 2008 and entitled Drug-Releasing Polyurethane Lacrimal Insert); U.S. Application Serial No. 12/378,710 (filed February 17, 2009 and entitled Lacrimal Implants and Related Methods); U.S. Application Serial No. 61/075,284 (filed June 24, 2008 and entitled Combination Treatment of Glaucoma); U.S. Application Serial No. 12/490,923 (filed June 24, 2009 and entitled Combination Treatment of Glaucoma); U.S. Application Serial No. 61/134,271 (filed July 8, 2008 and entitled Lacrimal Implant Body Including Comforting Agent); U.S. Application Serial No. 12/499,605 (filed July 8, 2009 and entitled Lacrimal Implant Body Including Comforting Agent); U.S. Application Serial No. 61/057,246 (filed May 30, 2008 and entitled Surface Treatment of Implants and Related Methods); U.S. Application Serial No. 61/132,927 (filed June 24, 2008 and entitled Surface Treated Implantable Articles and Related Methods); U.S. Application Serial No. 12/283,002 (filed September 5, 2008 and entitled Surface Treated Implantable Articles and Related Methods); U.S. Application Serial No. 12/231,989 (filed September 5, 2008 and entitled Lacrimal Implants and Related Methods); U.S. Application Serial No. 61/049,317 (filed April 30, 2008 and entitled Drug-Releasing Polyurethane Lacrimal Insert); U.S. Application Serial No. 12/231,986 (filed September 5, 2008 and entitled Drug Cores for Sustained Release of Therapeutic Agents); U.S. Application Serial No. 61/050,901 (filed May 6, 2008 and entitled Punctum Plug Detection); U.S. Application Serial No. 12/231,987 (filed September 5, 2008 and entitled Lacrimal Implant Detection); U.S. Application Serial No. 61/146,860 (filed January 23, 2009 and entitled Sustained Release Delivery of One or More Anti-Glaucoma Agents); U.S. Application Serial No. 61/152,909 (filed February 16, 2009 and entitled Sustained Release Delivery of One or More Anti-Glaucoma Agents); U.S. Application Serial No. 61/228,894 (filed July 27, 2009 and entitled Sustained Release Delivery of One or More Anti-Glaucoma Agents); U.S. Application Serial No. 61/277,000 (filed September 18, 2009 and entitled Drug Cores for Sustained Ocular Release of Therapeutic Agents); U.S. Application Serial No. 12/692,452 (filed January 22, 2010 and entitled Sustained Release Delivery of One or More Agents); U.S. Application Serial No. 61/283,100 (filed November 27, 2009 and entitled Lacrimal Implants Including Split and Insertable Drug Core); International Application Serial No. PCT/US2010/058129 (filed November 26, 2010, published as WO 2011/066479 and entitled Lacrimal Implants Including Split and Insertable Drug Core); U.S. Application Serial No. 61/139,456 (filed December 19, 2008 and entitled Substance Delivering Punctum Implants and Methods); U.S. Application Serial No. 12/643,502 (filed December 21, 2009 and entitled Substance Delivering Punctum Implants and Methods); U.S. Application Serial No. 10/825,047 (filed April 15, 2004 and entitled Drug Delivery via Punctal Plug); U.S. Application Serial No. 12/604,202 (filed October 22, 2009 and entitled Drug Delivery via Ocular Implant); International Application Serial No. PCT/US2005/023848 (filed July 1, 2005, published as WO 2006/014434 and entitled Treatment Medium Delivery Device and Methods for Delivery); International Application Serial No. PCT/US2007/065792 (filed April 2, 2007, published as WO 2007/115261 and entitled Drug Delivery Methods, Structures, and Compositions for Nasolacrimal System); and International Application Serial No. PCT/US2007/065789 (filed April 2, 2007, published as WO 2007/115259 and entitled Nasolacrimal Drainage System Implants for Drug Therapy).

In various embodiments of the methods of the invention, an implant including a retention structure is employed to retain the implant in the punctum or canaliculus. The retention structure is attached to or integral with the implant body. The retention structure comprises an appropriate material that is sized and shaped so that the implant can be easily positioned in the desired tissue location, for example, the punctum or canaliculus. In some embodiments, the drug core may be attached to the retention structure via, at least in part, the sheath. In some embodiments, the retention structure comprises a hydrogel configured to expand when the retention structure is placed in the punctum. The retention structure can comprise an attachment member having an axially oriented surface. In some embodiments, expansion of the hydrogel can urge against the axially oriented surface to retain the hydrogel while the hydrogel is hydrated. In some embodiments, the attachment member can comprise at least one of a protrusion, a flange, a rim, or an opening through a portion of the retention structure. In some embodiments, the retention structure includes an implant body portion size and shape to substantially match an anatomy of the punctum and canaliculus.

The retention structure may have a size suitable to fit at least partially within the canalicular lumen. The retention structure can be expandable between a small profile configuration suitable for insertion and a large profile configuration to anchor the retention structure in the lumen, and the retention structure can be attached near the distal end of the drug core. In specific embodiments, the retention structure can slide along the drug core near the proximal end when the retention structure expands from the small profile configuration to the large profile configuration. A length of the retention structure along the drug core can be shorter in the large profile configuration than the small profile configuration.

In some embodiments, the retention structure is resiliently expandable. The small profile may have a cross section of no more than about 0.2 mm, and the large profile may have a cross section of no more than about 2.0 mm. The retention structure may comprise a tubular body having arms separated by slots. The retention structure can be disposed at least partially over the drug core.

In some embodiments, the retention structure is mechanically deployable and typically expands to a desired cross-sectional shape, for example with the retention structure comprising a super elastic shape memory alloy such as Nitinol^{™}. Other materials in addition to Nitinol^{™} can be used, for example resilient metals or polymers, plastically deformable metals or polymers, shape memory polymers, and the like, to provide the desired expansion. In some embodiments polymers and coated fibers available from Biogeneral, Inc. of San Diego, CA may be used. Many metals such as stainless steels and non-shape memory alloys can be used and provide the desired expansion. This expansion capability permits the implant to fit in hollow tissue structures of varying sizes, for example canaliculae ranging from 0.3 mm to 1.2 mm (i.e. one size fits all). Although a single retention structure can be made to fit canaliculae from 0.3 to 1.2 mm across, a plurality of alternatively selectable retention structures can be used to fit this range if desired, for example a first retention structure for canaliculae from 0.3 to about 0.9 mm and a second retention structure for canaliculae from about 0.9 to 1.2 mm. The retention structure has a length appropriate to the anatomical structure to which the retention structure attaches, for example a length of about 3 mm for a retention structure positioned near the punctum of the canaliculus. For different anatomical structures, the length can be appropriate to provide adequate retention force, e.g. 1 mm to 15 mm lengths as appropriate.

Although the implant body may be attached to one end of the retention structure as described above, in many embodiments the other end of the retention structure is not attached to the implant body so that the retention structure can slide over the implant body including the sheath body and drug core while the retention structure expands. This sliding capability on one end is desirable as the retention structure may shrink in length as the retention structure expands in width to assume the desired cross-sectional width. However, it should be noted that many embodiments may employ a sheath body that does not slide in relative to the core.

In many embodiments, the retention structure can be retrieved from tissue. A projection, for example a hook, a loop, or a ring, can extend from a portion of the implant body to facilitate removal of the retention structure.

In some embodiments the sheath and retention structure can comprise two parts.

The lacrimal implants of the present invention have exceptional retention properties and are retained in the punctum and canaliculus for a period that is enhanced relative to a commercially available plug based upon the percentage of eyes in which an implant was implanted retaining the implant over a selected time period.

In an exemplary embodiment, the method of the invention uses a lacrimal implant configured to remain implanted in a punctum for at least about 1 week, 2 weeks, 3 weeks, 4 weeks, 5 weeks, 6 weeks, 7 weeks, 8, weeks, 9 weeks 10 weeks, 11 weeks, or at least about 12 weeks or more. In an exemplary embodiment, the lacrimal implant is configured to be retained by the puncta for the duration of the intended sustained release of the therapeutic agent. In various embodiments, the duration of the intended sustained release of the therapeutic agent is at least about 1 week, 2 weeks, 3 weeks, 4 weeks, 5 weeks, 6 weeks, 7 weeks, 8, weeks, 9 weeks 10 weeks, 11 weeks, or at least about 12 weeks or more. In various embodiments at least about 95%, at least about 90%, at least about 85% or at least about 80% of the implanted implants are retained for the duration of the intended controlled release of the therapeutic agent. In an exemplary embodiment, the implant is retained by the puncta for a length of time to show therapeutic efficacy.

In various embodiments, the present invention provides for the use of implants having structural features that enhance the retention of the implant in a punctum. Amongst other features, the heel of the present implant (e.g., **330**) is configured to come to rest in the lacrimal canaliculus ampulla (e.g., **252**), effectively locking the implant into place. However, the inventors have recognized that to prevent rotation and relative movement of the implanted device, which plays a role in the displacement of the device, a first member was needed to maintain the heel in the ampulla. Thus, the first member, e.g., **305**, is configured to stabilize the punctal plug within the lacrimal canaliculus, prevent rotation and maintain positioning of the plug when the surrounding tissue moves.

In an exemplary embodiment, the methods of the invention use an implant having an occlusive element. An occlusive element can be mounted to and expandable with the retention structure to inhibit tear flow. An occlusive element may inhibit tear flow through the lumen, and the occlusive element may cover at least a portion of the retention structure to protect the lumen from the retention structure. The occlusive element comprises an appropriate material that is sized and shaped so that the implant can at least partially inhibit, even block, the flow of fluid through the hollow tissue structure, for example lacrimal fluid through the canaliculus. The occlusive material may be a thin walled membrane of a biocompatible material, for example silicone, that can expand and contract with the retention structure. The occlusive element is formed as a separate thin tube of material that is slid over the end of the retention structure and anchored to one end of the retention structure as described above. Alternatively, the occlusive element can be formed by dip coating the retention structure in a biocompatible polymer, for example silicone polymer. The thickness of the occlusive element can be in a range from about 0.01 mm to about 0.15 mm, and often from about 0.05 mm to 0.1 mm.

***Methods of Use***

In embodiments, provided herein medical uses for delivering an ophthalmic drug to an eye for dry-eye treatment, comprising: placing a medical device disclosed herein comprising any one of the solid matrix sustained release ophthalmic formulations disclosed herein, on, in or near the eye of a patient, wherein the ophthalmic drug is cyclosporine. The medical device is a lacrimal implant, wherein the lacrimal implant is placed through a punctum and into a canalicular lumen of a patient.

In embodiments, treatment period for dry eye is about one month to about 6 months.

The methods of the present invention can be administered to a mammal in need of treatment by way of a variety of routes. For example, drug delivery systems may be used by implantation within a portion of the body in need of localized drug delivery, e.g., the interior portion of an eye. However, the exemplary matrix-controlled diffusion drug delivery systems may likewise be used in accordance with other surgical procedures known to those skilled in the field of ophthalmology. For example, the drug delivery systems can be administered to the region of the eye in need of treatment employing instruments known in the art, e.g., a flexible microcatheter system or cannula disclosed in U.S. Patent Application Publication No. 2002/0002362, or the intraretinal delivery and withdrawal systems disclosed in U.S. Pat. Nos. 5,273,530 and 5,409,457.

The pharmaceutically active agent may be released from the drug delivery device over a sustained and extended period of time. Optionally, the drug release rate may also be controlled through the attachment of an inert diffusion barrier by way of, for example, surface treatment of the drug delivery device. The surface treatment may be applied through a variety of surface treatment techniques known in the art, e.g., oxidative plasma, evaporative deposition, dip coating or extrusion techniques.

### Optional Formulation Components

The present formulation may further comprise a pharmaceutically acceptable carrier, e.g., excipients, suspending agents, diluents, fillers, salts, buffers, stabilizers, solubilizers, solvents, dispersion media, coatings, isotonic agents, and other materials known in the art. The pharmaceutical formulation optionally includes potentiators, complexing agents, targeting agents, stabilizing agents, cosolvents, pressurized gases, or solubilizing conjugates.

Exemplary excipients include sugars such as lactose, sucrose, mannitol, or sorbitol; cellulose preparations such as, maize starch, wheat starch, rice starch, potato starch, gelatin, gum tragacanth, methyl cellulose, hydroxypropylmethylcellulose, sodium caroxymethylcellulose, and/or polyvinylpyrrolidone (PVP). Preferred excipients include lactose, gelatin, sodium carboxymethyl cellulose, and low molecular weight starch products.

Exemplary suspending agents that can serve as valve lubricants in pressurized pack inhaler systems are desirable. Such agents include oleic acid, simple carboxylic acid derivatives, and sorbitan trioleate.

Exemplary diluents include water, saline, phosphate-buffered citrate or saline solution, and mucolytic preparations. Other diluents that can be considered include alcohol, propylene glycol, and ethanol; these solvents or diluents are more common in oral aerosol formulations. Physiologically acceptable diluents that have a tonicity and pH compatible with the alveolar apparatus are desirable. Preferred diluents include isotonic saline, phosphate buffered isotonic solutions whose tonicity have been adjusted with sodium chloride or sucrose or dextrose or mannitol.

Exemplary fillers include glycerin, propylene glycol, ethanol in liquid or fluid preparations. Suitable fillers for dry powder inhalation systems include lactose, sucrose, dextrose, suitable amino acids, and derivatives of lactose. Preferred fillers include glycerin, propylene glycol, lactose and certain amino acids.

Exemplary salts include those that are physiologically compatible and provide the desired tonicity adjustment. Monovalent and divalent salts of strong or weak acids are desirable. Preferred salts include sodium chloride, sodium citrate, ascorbates, sodium phosphates.

Exemplary buffers include phosphate or citrate buffers or mixed buffer systems of low buffering capacity. Preferred buffers include phosphate or citrate buffers.

### EXAMPLES

The following examples are put forth so as to provide those of ordinary skill in the art with a complete disclosure and description of how to use the embodiments provided herein and are not intended to limit the scope of the disclosure nor are they intended to represent that the Examples below are all of the experiments or the only experiments performed. Efforts have been made to ensure accuracy with respect to numbers used (e.g. amounts, temperature, etc.) but some experimental errors and deviations should be accounted for. Unless indicated otherwise, parts are parts by weight, and temperature is in degrees Centigrade. It should be understood that variations in the methods as described can be made without changing the fundamental aspects that the Examples are meant to illustrate.

### Example 1: Manufacturing of Cyclosporine sustained release formulation in a hydrophobic polymer and nonionic surfactant solid matrix

Cyclosporine, 1.0-1.3 dL/g intrinsic viscosity poly(caprolactone) (PCL) and polysorbate 80 (PS80) at the target weight ratio were dissolved via mixing at 50-60°C in a sufficient volume of tetrahydrofuran in a suitably sized round bottom flask. In this example, Cyclosporine was present from about 60 to 80% (w/w); polycaprolactone from about 14 to 32 % (w/w); and polysorbate 80 from about 4.5 to 22.5% (w/w). After dissolution, the round bottom flask (RBF) was installed on a rotary evaporator to remove the bulk THF. Residual THF was removed by storage in vacuum oven at 70-75°C, for a minimum of 19.5 hours at - 27inHg.

The mixture was injected into polyimide tubing (ID of 0.022 inches, OD of 0.024 inches) at a temperature of approximately 85°C to liquefy the mixture. The polyimide tubing filled with the Cyclosporine mixture was cooled to room temperature (e.g. 20 to 25°C). The filled tubing was cut into 0.95mm section lengths to form the drug core and the distal end was sealed with a small volume of UV cured methacrylate adhesive.

The glued drug cores were inserted glue dome first into the lumen of silicone punctal plugs in preparation for an elution rate testing procedure. For elution testing, the loaded punctal plugs were placed into individual 2 mL glass vials with screw-on caps. About 1.0mL of elution buffer comprised of 0.1% by weight sodium dodecyl sulfate detergent dissolved in phosphate-buffered saline. The vial was then capped and mixed at 100 rpm in a shaker-incubator at 30-35°C. After one day of mixing, the vial was removed, the plug transferred to a fresh vial of elution buffer for the day 2 incubation. The cyclosporine-containing day 1 elution buffer was retained for analysis for cyclosporine content. Serial transfers and incubation of the plug in elution buffer were performed on at least the following target days: 1, 2, 3, 4, 7, 8, 9, 10, 11, 14, 21, 28, 35, 42, 49, 56 and 63. Recorded times and dates for the shaking start times and removal times were used to calculate exact mixing times. The spent plugs at the end of elution testing were retained for analytical determination of their residual cyclosporine contents.

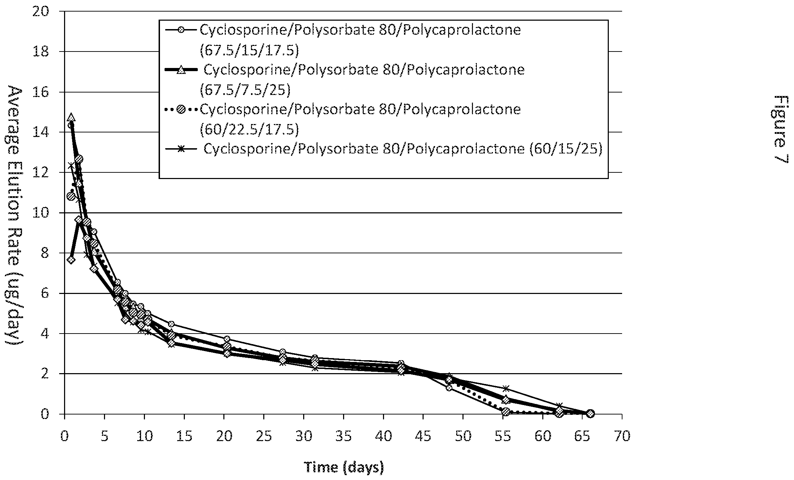

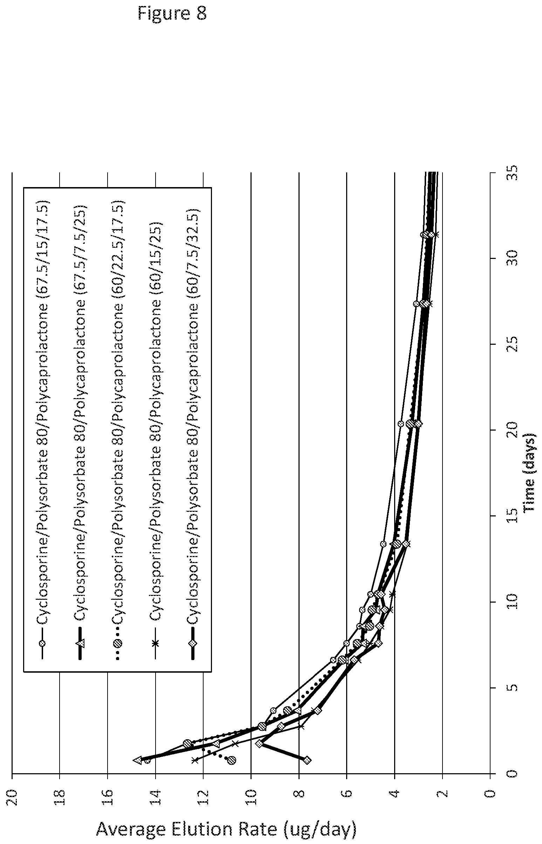

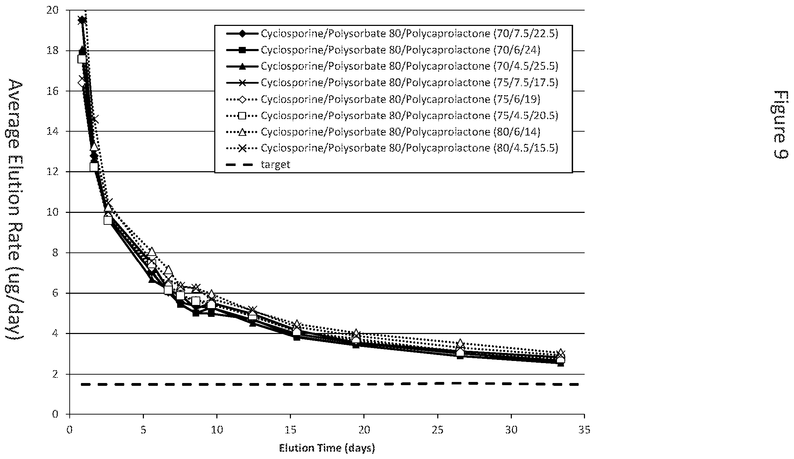

The daily elution rate in µg/mL per day were calculated using the analytically determined cyclosporine concentrations and mixing times for each vial sample. Average daily elution rates were calculated from multiple vials derived from insert samples taken from each extrusion, or section thereof. *See* Figures 7 to 9, wherein the elution was measured from drug cores comprising different ratios of cyclosporine/PS80/PCL. The target elution rate per day, as shown in Figure 9, is 1.5 µg/day.

### Example 2: Manufacturing of Cyclosporine sustained release formulation in one or more hydrophobic polymer solid matrix

Cyclosporine, 1.0-1.3 dL/g intrinsic viscosity poly(caprolactone) (PCL) and polyvinyl acetate (PVAc) at the target weight ratio were dissolved via mixing at 50-60°C in a sufficient volume of tetrahydrofuran in a 20mL vial. In this example, Cyclosporine was present from 70% to 80% (w/w); polycaprolactone from 15 to 25% (w/w); and PVAc from about 0 to 15% (w/w). After dissolution, the solution was transferred to a suitably sized round bottom flask (RBF) and the RBF was installed on a rotary evaporator to remove the bulk THF. Residual THF was removed by storage in vacuum oven at 70-80°C, for a minimum of 19.5 hours at -27inHg.

The mixture was injected into polyimide tubing (ID of 0.022 inches, OD of 0.024 inches) at a temperature of between approximately 90°C to 110 °C to liquefy the mixture. The polyimide tubing filled with the Cyclosporine mixture was cooled to room temperature (e.g. 20 to 25°C). The filled tubing was cut into sections from 0.95 to 1.10mm lengths at an elevated temperature of between approximately 60°C to 70 °C to form the drug core and the distal end was sealed with a small volume of UV cured methacrylate adhesive.

The glued drug cores were inserted glue dome first into the lumen of silicone punctal plugs in preparation for an elution rate testing procedure. For elution testing, the loaded punctal plugs were placed into individual 2 mL glass vials with screw-on caps. About 1.0mL of elution buffer comprised of 0.1% by weight sodium dodecyl sulfate detergent dissolved in phosphate-buffered saline. The vial was then capped and mixed at 100 rpm in a shaker-incubator at 30-35°C. After one day of mixing, the vial was removed, the plug transferred to a fresh vial of elution buffer for the day 2 incubation. The cyclosporine-containing day 1 elution buffer was retained for analysis for cyclosporine content. Serial transfers and incubation of the plug in elution buffer were performed on at least the following target days: 1, 2, 3, 4, 7, 8, 9, 10, 11, 14, 21, 28, 35, 42, 49, 56 and 63. Recorded times and dates for the shaking start times and removal times were used to calculate exact mixing times. The spent plugs at the end of elution testing were retained for analytical determination of their residual cyclosporine contents.

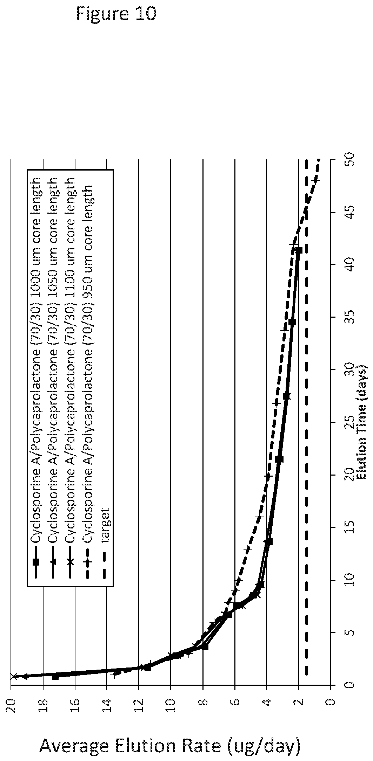

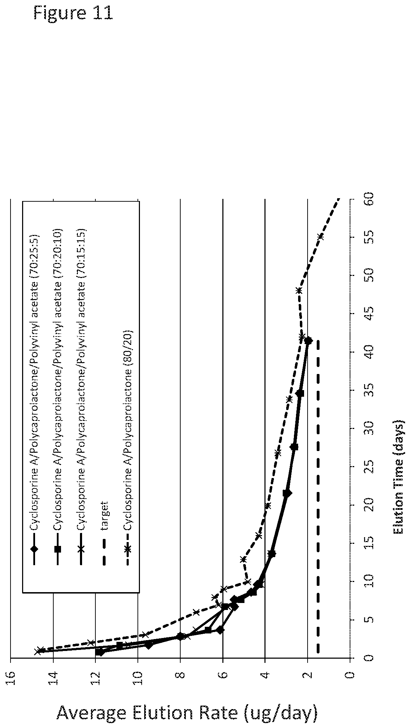

The daily elution rate in µg/mL per day were calculated using the analytically determined cyclosporine concentrations and mixing times for each vial sample. Average daily elution rates were calculated from multiple vials derived from insert samples taken from each extrusion, or section thereof. *See* Figures 10 and 11, wherein the elution was measured from drug cores comprising different ratios of cyclosporine/PCL/polyvinyl acetate. The target elution rate per day, as shown in Figure 10 and 11, is 1.5 µg/day.

### Example 3: Manufacturing of Cyclosporine sustained release formulation in one or more hydrophobic polymer solid matrix with or without a surfactant

Cyclosporine, 1.0-1.3 dL/g intrinsic viscosity poly(caprolactone) (PCL), polyvinyl acetate (PVAc) and polysorbate 80 (PS80) at the target weight ratio were dissolved via mixing at 50-60°C in a sufficient volume of tetrahydrofuran in a 20mL vial. In this example, Cyclosporine was present at 70 or 75% (w/w); polycaprolactone from 5 to 20% (w/w); PVAc from about 5 to 20% (w/w); and PS80 from about 0 to 3%. After dissolution, the solution was transferred to a suitably sized round bottom flask (RBF) and the RBF was installed on a rotary evaporator to remove the bulk THF. Residual THF was removed by storage in vacuum oven at 70-80°C, for a minimum of 16.5 hours at -27inHg.

The mixture was injected into polyimide tubing (ID of 0.022 inches, OD of 0.024 inches) at a temperature of between approximately 90°C to 110 °C to liquefy the mixture. The polyimide tubing filled with the Cyclosporine mixture was cooled to room temperature (e.g. 20 to 25°C). The filled tubing was cut into sections of 1.10mm lengths at an elevated temperature of between approximately 60°C to 70 °C to form the drug core and the distal end was sealed with a small volume of UV cured methacrylate adhesive.

The glued drug cores were inserted glue dome first into the lumen of silicone punctal plugs in preparation for an elution rate testing procedure. For elution testing, the loaded punctal plugs were placed into individual 2 mL glass vials with screw-on caps. About 1.0mL of elution buffer comprised of 0.1% by weight sodium dodecyl sulfate detergent dissolved in phosphate-buffered saline. The vial was then capped and mixed at 100 rpm in a shaker-incubator at 30-35°C. After one day of mixing, the vial was removed, the plug transferred to a fresh vial of elution buffer for the day 2 incubation. The cyclosporine-containing day 1 elution buffer was retained for analysis for cyclosporine content. Serial transfers and incubation of the plug in elution buffer were performed on at least the following target days: 1, 2, 3, 4, 7, 8, 9, 10, 11, 14, 21, 28, 35, 42, 49, 56 and 63. Recorded times and dates for the shaking start times and removal times were used to calculate exact mixing times. The spent plugs at the end of elution testing were retained for analytical determination of their residual cyclosporine contents.

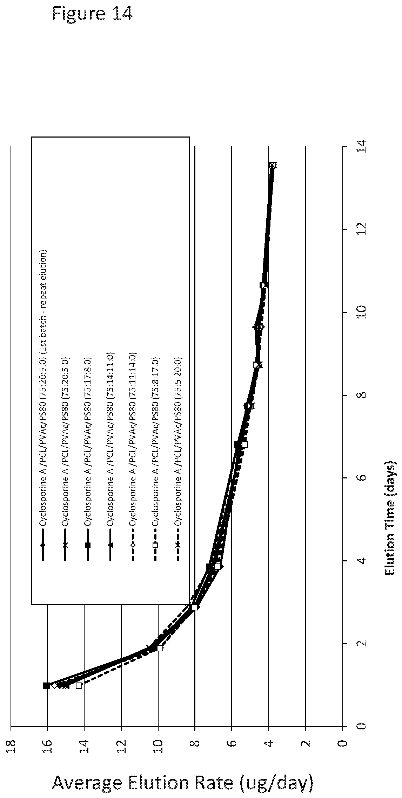

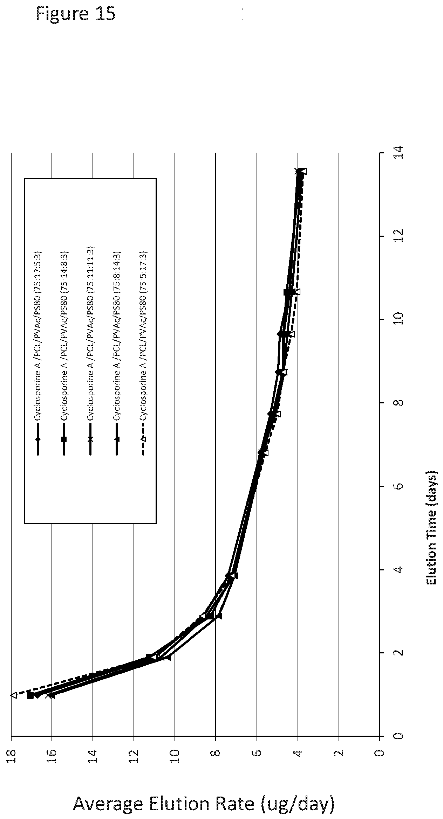

The daily elution rate in µg/mL per day were calculated using the analytically determined cyclosporine concentrations and mixing times for each vial sample. Average daily elution rates were calculated from multiple vials derived from insert samples taken from each extrusion, or section thereof. *See* Figures 12 to 15, wherein the elution was measured from drug cores comprising different ratios of cyclosporine/PCL/polyvinyl acetate. The target elution rate per day, as shown in Figure 12 and 13, is 1.5 µg/day.

## Claims

1. A sustained release ophthalmic formulation for topical delivery of an ophthalmic drug, comprising:
cyclosporine admixed with one or more hydrophobic polymers to form a solid matrix composition, wherein the composition is in the form of a drug core and configured for placement within a lacrimal canaliculus; and wherein the drug core does not comprise one or more of silicone,
or methacrylate polymers or monomers; wherein a first hydrophobic polymer is polycaprolactone and is present from 5 to 30% w/w, a second hydrophobic polymer is polyvinyl acetate and is present from 0 to 20% w/w, and the cyclosporine is present from 70 to 80% w/w.

2. The formulation of claim 1, wherein the drug core does not comprise one or more of: a nonionic surfactant selected from tyloxapol, a sorbitan ester, polyoxyethylene ethers, a polysorbate or a combination thereof; PEG polymers; a hydrophilic polymer selected from polyethylene glycol (PEG) polymers, acrylate-derivatized PEG (PEGDA) polymers, polysaccharide polymers, hydrophilic polyanhydrides or a combination thereof.

3. A lacrimal implant comprising:
a punctal plug comprising a plug body and a drug insert, wherein the insert comprises;
a drug core comprising the formulation according to any one of claim 1-2 and an impermeable sheath body partially covering the drug core, wherein the sheath body is configured to provide an exposed proximal end of the drug core in direct contact with tear fluid that releases an ophthalmic drug to the eye when the drug insert is disposed within a channel of the punctal plug and the punctal plug is suitable to be inserted into the lacrimal canaliculus of a patient.

4. A lacrimal implant of claim 3 for use in the treatment of dry eye.

## Patentansprüche

1. Ophthalmische Formulierung zur verzögerten Freisetzung zur topischen Abgabe eines ophthalmischen Arzneimittels, umfassend:
Cyclosporin vermischt mit einem oder mehreren hydrophoben Polymeren, um eine feste Matrixzusammensetzung zu bilden, wobei die Zusammensetzung in der Form eines Arzneimittelkerns und für Platzierung innerhalb eines Tränenkanälchens konfiguriert ist; und wobei der Arzneimittelkern nicht eines oder mehrere von Silikon oder Methacrylatpolymeren oder -monomeren umfasst; wobei
ein erstes hydrophobes Polymer Polycaprolacton ist und von 5 bis 30 Gew.-% vorhanden ist, ein zweites hydrophobes Polymer Polyvinylacetat ist und von 0 bis 20 Gew.-% vorhanden ist und das Cyclosporin von 70 bis 80 Gew.-% vorhanden ist.

2. Formulierung nach Anspruch 1, wobei der Arzneimittelkern nicht eines oder mehrere von Folgendem umfasst:
einem nichtionischen Tensid ausgewählt aus Tyloxapol, einem Sorbitanester, Polyoxyethylenethern, einem Polysorbat oder einer Kombination davon; PEG-Polymeren; einem hydrophilen Polymer ausgewählt aus Polyethylenglykol(PEG-)Polymeren, Acrylat-derivatisierten PEG(PEGDA-)Polymeren, Polysaccharidpolymeren, hydrophilen Polyanhydriden oder einer Kombination davon.

3. Tränenimplantat, umfassend:
einen Pünktchenstöpsel, der einen Stöpselkörper und einen Arzneimitteleinsatz umfasst, wobei der Einsatz Folgendes umfasst;
einen Arzneimittelkern, der die Formulierung nach einem der Ansprüche 1-2 umfasst, und
einen undurchlässigen Hüllenkörper, der den Arzneimittelkern teilweise bedeckt, wobei der Hüllenkörper konfiguriert ist, um ein exponiertes proximales Ende des Arzneimittelkerns in direktem Kontakt mit Tränenflüssigkeit bereitzustellen, das ein ophthalmisches Arzneimittel an das Auge freisetzt, wenn der Arzneimitteleinsatz innerhalb eines Kanals des Pünktchenstöpsels angeordnet ist und der Pünktchenstöpsel geeignet ist, um in das Tränenkanälchen eines Patienten eingesetzt zu werden.

4. Tränenimplantat nach Anspruch 3 zur Verwendung bei der Behandlung von trockenem Auge.

## Revendications

1. Formulation ophtalmique à libération prolongée pour l'administration topique d'un médicament ophtalmique, comprenant :
de la cyclosporine mélangée à un ou plusieurs polymères hydrophobes pour former une composition de matrice solide, la composition étant sous la forme d'un noyau de médicament et conçue pour être placée dans un canalicule lacrymal ; et le noyau de médicament ne comprenant pas un ou plusieurs parmi la silicone, ou des polymères ou des monomères de méthacrylate ; dans laquelle
un premier polymère hydrophobe est la polycaprolactone et est présent à raison de 5 à 30 % p/p, un second polymère hydrophobe est l'acétate de polyvinyle et est présent à raison de 0 à 20 % p/p, et la cyclosporine est présente à raison de 70 à 80 % p/p.

2. Formulation de la revendication 1, le noyau de médicament ne comprenant pas un ou plusieurs :
d'un tensioactif non ionique choisi parmi le tyloxapol, un ester de sorbitan, des éthers de polyoxyéthylène, un polysorbate ou une combinaison de ceux-ci ; de polymères PEG ; d'un polymère hydrophile choisi parmi des polymères de polyéthylène glycol (PEG), des polymères PEG dérivés d'acrylate (PEGDA), des polymères de polysaccharides, des polyanhydrides hydrophiles ou une combinaison de ceux-ci.

3. Implant lacrymal comprenant :
un bouchon méatique comprenant un corps de bouchon et un insert de médicament, dans lequel l'insert comprend ;
un noyau de médicament comprenant la formulation selon l'une quelconque des revendications 1 et 2 et
un corps de gaine imperméable recouvrant partiellement le noyau de médicament, dans lequel le corps de gaine est conçu pour fournir une extrémité proximale exposée du noyau de médicament en contact direct avec le liquide lacrymal qui libère un médicament ophtalmique dans l'œil lorsque l'insert de médicament est disposé à l'intérieur d'un canal du bouchon méatique et le bouchon méatique peut être inséré dans le canalicule lacrymal d'un patient.

4. Implant lacrymal de la revendication 3 destiné à être utilisé dans le traitement de l'œil sec.
